(19) Europäisches Patentamt
European Patent Office
Office européen des brevets

(11) **EP 4 082 498 A1**

(12) **EUROPEAN PATENT APPLICATION**
published in accordance with Art. 153(4) EPC

(43) Date of publication:
**02.11.2022 Bulletin 2022/44**

(21) Application number: **20904432.0**

(22) Date of filing: **23.12.2020**

(51) International Patent Classification (IPC):
**A61F 13/532** (2006.01)    **A61F 13/534** (2006.01)

(52) Cooperative Patent Classification (CPC):
**A61F 13/532; A61F 13/534**

(86) International application number:
**PCT/JP2020/048177**

(87) International publication number:
**WO 2021/132338 (01.07.2021 Gazette 2021/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.12.2019 PCT/JP2019/050269**

(71) Applicant: **Kao Corporation
Chuo-ku
Tokyo 103-8210 (JP)**

(72) Inventor: **KURAMAE, Ryota
Haga-gun, Tochigi 321-3497 (JP)**

(74) Representative: **Vossius & Partner
Patentanwälte Rechtsanwälte mbB
Siebertstraße 3
81675 München (DE)**

(54) **ABSORBENT SHEET, ABSORBENT ARTICLE, AND ABSORBENT-SHEET MANUFACTURING METHOD**

(57) An absorbent sheet (1) includes a first fiber sheet (11), a second fiber sheet (12), and an absorbent polymer (13) disposed between the fiber sheets (11, 12), both of the fiber sheets (11, 12) being joined to each other by an adhesive (15). In the absorbent sheet (1), in a region where the absorbent polymer (13) is disposed, the absorbent polymer (13) is disposed in such a manner that no macroscopically recognizable gap is observed. The region includes a portion (17) where both the fiber sheets (11, 12) are directly joined to each other only by the adhesive (15) without intervention of the absorbent polymer (13).

Fig. 2

**Description**

Technical Field

The present invention relates to an absorbent sheet, an absorbent article, and a producing method of the absorbent sheet.

Background Art

For the purpose of improving liquid absorbency, a sheet-like material in which an absorbent polymer is disposed between two sheets, and an absorbent article containing the sheet-like material have been developed. For example, Patent Literature 1 discloses a water-absorbent water-resistant sheet having three-layer structure including a hydrophobic nonwoven fabric having water-resistance of at least 150 mmH$_2$O, a hydrophilic sheet having moisture diffusiveness, and an SAP layer interposed between the hydrophobic nonwoven fabric and the hydrophilic sheet and made of SAP particles having an average particle size of 500 $\mu$m or less.

Patent Literature 2 discloses a water-absorbent structure having a structure in which a water-absorbent layer containing a water-absorbent resin and an adhesive is sandwiched with nonwoven fabric from an upper side and a lower side of the water-absorbent layer, wherein the content of the water-absorbent resin is 100 to 1000 g/m$^2$, a mass average particle size of the water-absorbent resin is 50 to 800 $\mu$m, a particle size rate index of the water-absorbent resin 0.12 s/$\mu$m or less, and a peeling strength of the water-absorbent sheet structure is 0.05 to 3.0 N/7 cm.

Further, Patent Literature 3 discloses an absorbent article in which an absorbent member is interposed between a liquid-permeable topsheet and a liquid-impermeable backsheet, wherein the absorbent member includes an upper-layer absorbent member made of pulp and a super absorbent polymer and a lower-layer absorbent member which is disposed adjacent to a non-skin side of the upper absorbent member and includes a super absorbent polymer disposed between two sheets, an opening extending in a longitudinal direction is formed in the upper-layer absorbent member, and a depression groove is formed on a skin-facing surface of the absorbent article by depressing the water-permeable topsheet in the opening.

Patent Literature 4 discloses a disposable absorbent article includes an absorbent mat provided between a liquid-permeable topsheet and a liquid-impermeable backsheet, the absorbent mat including a sheet-like water-absorbent layer that contains a water-absorbent resin powder but does not contain a pulp fiber. Further, the sheet-like water-absorbent layer is disclosed in Patent Literature 4 in which a plurality of water-absorbent resin powder existence regions containing water-absorbent resin powders and water-absorbent resin powder non-existence regions are formed adjacent to each other between nonwoven fabric sheets.

Citation List

Patent Literature

Patent Literature 1: JP2002-325799A

Patent Literature 2: US 2012/328861 A1

Patent Literature 3: JP2018-50987A

Patent Literature 4: US 2007/093164 A1

Summary of Invention

The present invention is related to an absorbent sheet including a first fiber sheet, and a second fiber sheet, and an absorbent polymer disposed between the first fiber sheet and the second fiber sheet.

In the present embodiment, the first fiber sheet and the second fiber sheet are joined to each other by an adhesive.

In the absorbent sheet of the present embodiment, in a region where the absorbent polymer is disposed, the absorbent polymer is disposed in such a manner that no macroscopically recognizable gap is observed.

In the absorbent sheet of the present embodiment, the region includes a portion where both the first fiber sheet and the second fiber sheet are directly joined to each other only by the adhesive without intervention of the absorbent polymer.

The present invention also relates to an absorbent article including the absorbent sheet.

Further, the present invention also relates to a method for producing the absorbent sheet.

The producing method of the present embodiment includes applying the adhesive to each of one surface of the fiber sheet and one surface of the second fiber sheet, and spraying the absorbent polymer to an application surface of the adhesive on the second fiber sheet.

The producing method of the present embodiment further includes superposing the fiber sheets on each other such that application surfaces of the adhesive on the respective fiber sheets face each other.

Brief Description of Drawings

[Fig. 1] Fig. 1 is a schematic cross-sectional view showing an absorbent sheet according to an embodiment of the present invention.

[Fig. 2] Fig. 2 is a schematic perspective view showing the absorbent sheet according to the embodiment of the present invention.

[Fig. 3] Fig. 3 is a schematic cross-sectional view showing an absorbent sheet according to another embodiment of the present invention.

[Fig. 4] Fig. 4 is a schematic view showing a producing apparatus used for producing the absorbent sheet according to an embodiment.

Description of Embodiments

As disclosed in Patent Literatures 1 to 3 described above, since the sheet-like material including the absorbent polymer disposed between two sheets is used to prevent unintended movement or uneven distribution of the absorbent polymer, the absorbent polymer is often strongly sandwiched or firmly joined between two sheets. In this case, the absorbent polymer cannot swell sufficiently at the time of liquid absorption, resulting in reducing liquid absorption performance. On the other hand, when the absorbent polymer is weakly sandwiched or weakly joined between two sheets, the absorbent polymer will unintentionally move or unevenly distribute in a surface direction of the sheet, whereby intended liquid absorption performance cannot be exhibited, and as a result, adverse effects such as liquid leakage of the excreted liquid and a liquid return to a wearer's skin side may occur. No studies have been made on the techniques disclosed in Patent Literatures 1 to 3 to solve these problem, and improvements has been desired.

In the technique disclosed in Patent Literature 4 described above, since the region containing no absorbent polymer is formed in a state of being macroscopically recognized, the absorbent polymer unintentionally moves or unevenly distributes in the region. As a result, not only intended liquid absorption performance cannot be exhibited, but also unevenness is likely to occur after the absorbent polymer swells, which gives a user a sense of discomfort. Patent Literature 4 also discloses a technique in which a region containing no absorbent polymer is fused, but in this case, a space cannot be secured in which the absorbent polymer cannot sufficiently swell, resulting in reducing liquid absorption performance.

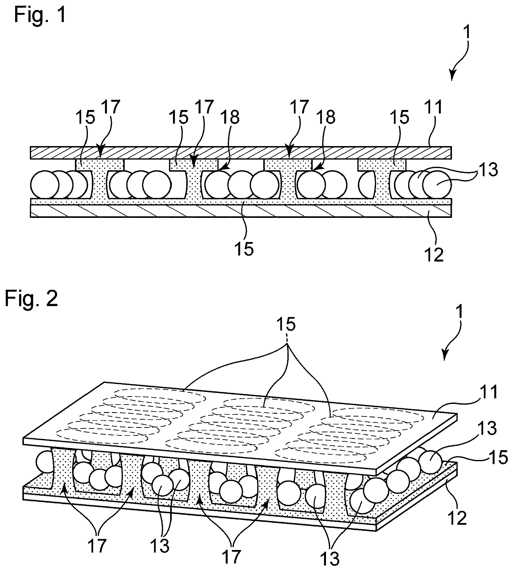

A preferred embodiment of the present invention will be described below with reference to the drawings. Fig. 1 shows an embodiment of an absorbent sheet of the present invention. An absorbent sheet 1 shown in Fig. 1 includes a first fiber sheet 11, a second fiber sheet 12, and a plurality of particles of an absorbent polymer 13 disposed between both of the fiber sheets 11 and 12. The first fiber sheet 11 and the second fiber sheet 12 are joined to each other of the adhesive 15. In the embodiment shown in Fig. 1, the adhesive 15 is disposed on a surface on each of the fiber sheets 11 and 12 facing the absorbent polymer 13. In the embodiment shown in Fig. 1, the adhesive 15 or other members do not exist on any of the outer surfaces of the first fiber sheet 11 and the second fiber sheet 12, but as will be described below, a fiber-entangling layer and other members of constituent members of the absorbent article may be disposed on the outer surface of each of the fiber sheets 11 and 12 without impediment.

When the absorbent sheet 1 is seen in a plan view, in a region where the absorbent polymer 13 is disposed, the absorbent polymer 13 is disposed in such a manner that no macroscopically recognizable gap is observed. The "no macroscopically recognizable gap is observed" means that the absorbent polymer 13 is arranged so as to cover one surface of the fiber sheet uniformly when the region sprayed with the absorbent polymer 13 is seen with a naked eye, but voids between the absorbent polymers 13 are permitted to be unintentionally formed when the region is seen microscopically. The size of the void is about 10 to 1000 $\mu$m. In the following description, the void between the absorbent polymers 13, that is microscopically observed in the region sprayed with the absorbent polymer 13, is also referred to as a "microscopic void".

The region sprayed with the absorbent polymer 13 includes portions 17 (hereinafter, simply referred to as "directly joined portions 17") where the first fiber sheet 11 and the second fiber sheet 12 are directly joined by the adhesive 15 without intervention of the absorbent polymer 13. The directly joined portions 17 are formed in the microscopic voids described above, respectively. When the directly joined portion 17 is viewed in cross section in a sheet thickness direction, the adhesive 15 becomes a columnar shape, and both of the fiber sheets 11 and 12 are directly joined to each other. Further, as shown in Fig. 2, the plurality of directly joined portions 17 are formed in a form of regularly or irregularly scattered dots when the absorbent sheet 1 is viewed in a sheet plane direction. Since the directly joined portions 17 are formed, it is possible to sufficiently exhibit liquid absorbency of the absorbent polymer 13 while retaining the absorbent polymer 13 at a predetermined position of the absorbent sheet 1. The directly joined portions 17 can be formed by appropriately adjusting a basis weight or a particle size of the absorbent polymer 13 or the applied amount and an area of the adhesive 15 which will be described below, for example.

As shown in Figs. 1 and 2, the absorbent sheet 1 preferably includes not only the directly joined portions 17 but also portions 18 (hereinafter, simply referred to as "indirectly joined portions 18") where both of the fiber sheets 11 and 12 are joined to each other by the adhesive 15 through the absorbent polymer 13. When the absorbent sheet 1 is viewed in cross section, in the indirectly joined portion 18, an application portion of the adhesive 15 on the first fiber sheet 11, an existence portion of the absorbent polymer 13, and an application portion of the adhesive 15 on the second fiber sheet 12 overlap with each other in a thickness direction. With such a configuration, it is possible to retain the absorbent polymer 13 at a predetermined position of the absorbent sheet 1, to further reduce unintended movement or uneven distribution of the absorbent polymer 13, and to further improve the liquid absorbency of the absorbent sheet 1.

As the adhesive 15, it is preferable to use an adhesive having stretchable flexibility depending on a swelling change due to liquid absorption of the absorbent polymer 13. Examples of such raw materials include a rubber-based adhesive including: an acrylic adhesive containing one or more (co) polymers of vinyl monomers (for example, an ethylene vinyl acetate copolymer) such as 2-ethylhexyl acrylate, butyl acrylate, ethyl acrylate, cyanoacrylate, vinyl acetate, and methyl methacrylate; a silicone-based adhesive containing a polydimethylsiloxane polymer; a natural rubber-based adhesive containing natural rubber; an isoprene-based adhesive containing one or more of polyisoprene and chloroprene; and a styrene-based adhesive containing one or more of Styrene-butadiene copolymer (SBR), styrene-isoprene-styrene block copolymer (SIS), styrene-butadiene-styrene block copolymer (SBS), styrene-ethylene-butadiene-styrene block copolymer (SEBS), and styrene-ethylene-propylene-styrene block copolymer (SEPS). These materials may be used alone or in combination of two or more. As shown in Fig. 1, when the adhesives 15 are disposed on surfaces of the fiber sheets 11 and 12 facing the absorbent polymer 13, respectively, the adhesive disposed on the surface of the first fiber sheet 11 and the adhesive disposed on the second fiber sheet may be the same type or different types.

Among these adhesives, the rubber-based adhesives may be preferably used as the adhesive 15 from the viewpoint of being excellent in flexibility and elasticity, maintaining a state where the fiber sheets 11 and 12 are directly joined to each other even after the swelling of the absorbent polymer, and expressing a shrinkage force to easily retain the absorbent polymer 13 between both of the fiber sheets 11 and 12, and the styrene-based adhesive is more preferably used among the rubber-based adhesives.

The adhesive 15 is preferably the hot-melt adhesive from the viewpoint of achieving both flexibility of the adhesive and adhesiveness to the sheet. Examples of the hot-melt adhesive include an adhesive obtained by adding tackifiers such as petroleum resin or polyterpene resin, plasticizers such as paraffin oil, and, if necessary, a phenol-based antioxidant, an amine-based antioxidant, a phosphorus-based antioxidant, and a benzimidazole-based antioxidant to various adhesives described above.

A relaxation time of the adhesive 15 obtained by viscoelasticity measurement is preferably 1 second or longer, more preferably 2 seconds or longer, and even more preferably 3 seconds or longer, as well as preferably 20 seconds or shorter, more preferably 10 seconds or shorter, and even more preferably 5 seconds or shorter at 50°C. When the adhesive 15 has such a value, it is possible to express appropriate flexibility and elasticity, and to retain the absorbent polymer in a state where the fiber sheets 11 and 12 are directly joined to each other even after the swelling of the absorbent polymer.

In general, the relaxation time is a physical property indicating the degree of fluidity of the material; when the relaxation time is short, the adhesive easily flows, so a part stretched by a surface tension acting when the adhesive is stretched is thinned and such a part is easily fractured. As a result, the joined state between the fiber sheets 11 and 12 may not be maintained. As a result of diligent study in this regard, the inventor has found the adhesive used preferably in the present invention that a correlation between the relaxation time at 50°C and the extensibility with the adhesive is high, and as the relaxation time at 50°C becomes longer, the stretched part caused by the stretching of the adhesive is hard to be fractured.

The relaxation time of the adhesive 15 at 50°C obtained by the viscoelasticity measurement is calculated as a reciprocal number of a value of $\tan\theta$ obtained when dynamic viscoelasticity of the adhesive 15 is measured under the following conditions. Specifically, using a rotary rheometer (a model "Physica MCR301" manufactured by Anton Paar Co.), the adhesive 15 to be measured is interposed between a circular backing plate in a plan view that supports the measurement sample from below and a circular pressing plate in a plan view that is disposed above and facing the backing plate. In this state, the adhesive 15 is circular in shape in a plan view, and has a thickness of 1.5 mm and a diameter of 12 mm. The dynamic viscoelasticity of the adhesive 15 is measured in a temperature range from 120°C to - 10°C under conditions that a frequency at the time of measurement is set to 1 Hz, a strain amplitude is set to 0.05%, and a cooling rate is set to 2°C/min. The tanθ is a value obtained by dividing a loss modulus G" by a storage modulus G'.

When the adhesive 15 to be measured is a component of the absorbent sheet 1, the adhesive is collected from the absorbent sheet by a solvent extraction method to be described below, and the collected adhesive is a measurement target for the viscoelasticity measurement described above. Further, when the adhesive 15 to be measured is not a component of the absorbent sheet 1 and is unused, the unused adhesive is used as it is as a measurement target. The relaxation time of the adhesive may be within the above-described range in at least one case regardless of whether the adhesive is a component of the absorbent sheet 1.

The solvent extraction method of the adhesive can be performed by the following method, for example. First, the absorbent sheet 1 including the adhesive 15 is mixed with a solvent capable of dissolving the adhesive 15 in a container such as a beaker, and the adhesive 15 is dissolved in the solvent. Next, an adhesive solution is collected by separation of a solid body such as the fiber sheet and an adhesive solution, the adhesive solution is dried under reduced pressure using a rotary evaporator to obtain a solid adhesive. The adhesive obtained in such a manner is used as a measurement target in the measurement of the relaxation time. The solvent used for dissolving the adhesive may be appropriately selected depending on a type of the adhesive. When the adhesive to be measured is a hot-melt adhesive, one or more of toluene, methyl ethyl ketone, and heptane can be used as the solvent used for dissolving the adhesive.

The fiber sheet used in the present invention is a fiber aggregate having a thickness of 5 mm or less measured under a pressure of 1.7 kPa. The thickness of the fiber sheet can be measured by a laser displacement meter, for example. Examples of constituent fibers of each of the fiber sheets 11 and 12 include wood pulp, natural pulp such as cotton and hemp, modified pulp such as mercerized pulp and chemically cross-linked pulp, and various fibers, for example, synthetic fibers composed of a resin such as polyethylene and polypropylene. The form of each of the fiber sheets 11 and 12 is paper, woven fabric, or nonwoven fabric.

In the absorbent sheet having the above configuration, an appropriate joining force can be expressed between the fiber sheets to the extent that the absorbent polymer does not move or fall off due to the adhesive existing on the directly joined portion, and the absorbent polymer can be supported at an appropriate position between the fiber sheets. Further, since the absorbent polymer expresses the appropriate joining force to the extent that it can sufficiently swell, the liquid absorbency of the absorbent polymer can be sufficiently exhibited. According to a preferred aspect of the present invention, since the adhesive has elasticity, stretching of the adhesive existing at the directly joined portion due to the swelling of the absorbent polymer and the resulting shrinkage of the adhesive are balanced in force, and it is possible to efficiently express the joining force capable of realizing both the support of the absorbent polymer at the appropriate position and securement of a space where the absorbent polymer can swell. As a result, the liquid absorbency of the absorbent sheet is further improved.

From the viewpoint of making the above-described effects remarkable, a storage modulus G' at 25°C of the adhesive 15 is preferably 10000 Pa or more, and more preferably 50000 Pa or more. Further, a loss modulus G" at 25°C of the adhesive 15 is preferably 10000 Pa or more, and more preferably 50000 Pa or more. When the adhesive 15 has such values, it is possible to more efficiently express a holding force capable of realizing both the support of the absorbent polymer at the appropriate position and securement of a space where the absorbent polymer can swell. The storage modulus G' and the loss modulus G" can be measured by, for example, the same method as the viscoelasticity measurement described above. The storage modulus G' and the loss modulus G" can be adjusted by appropriate change of a composition and a molecular weight of a base polymer contained in the adhesive 15 and the content of a plasticizer.

The adhesives 15 preferably exist on the surfaces of the fiber sheets 11 and 12 facing the absorbent polymer 13 and in the voids between fibers constituting the fiber sheets 11 and 12. With such a configuration, peeling hardly occurs at an interface between each of the fiber sheets 11 and 12 and the adhesive 15, and as a result, the absorbent polymer 13 can be retained for a long time by an elastic force of the adhesive 15 existing at the directly joined portion 17. In order to confirm that the adhesive 15 exists in the voids between the fibers constituting each of the fiber sheets 11 and 12, the fiber sheet to be measured is cut to a predetermined size, it can be confirmed by observing a cross section thereof with a scanning electron microscope (for example, a scanning electron microscope JCM-6000 produced by JEOL Ltd.) at a magnification of 50 to 500 times.

The adhesive 15 is preferably applied to the surface of the first fiber sheet 11 facing the absorbent polymer 13 and the surface of the second fiber sheet 12 facing the absorbent polymer 13. With such a configuration, the adhesives applied to the fiber sheets 11 and 12 are bonded to each other to efficiently form the directly joined portion 17 on the absorbent sheet 1 and to realize both the support of the absorbent polymer 13 at the appropriate position and the securement of the space where the absorbent polymer 13 can swell. Additionally, since the adhesives 15 can easily exist in the voids between the fibers constituting the fiber sheets 11 and 12, there is also an advantage that peeling hardly occurs at the interfaces between each of the fiber sheets 11 and 12 and the adhesive 15.

As shown in Figs. 1 and 2, the application area of the adhesive 15 on the second fiber sheet 12 is preferably larger than the application area of the adhesive 15 on the first fiber sheet 11. Additionally, focusing on the portions of the fiber sheets 11 and 12 to which the adhesive 15 is applied, a first basis weight of the adhesive 15 applied to the first fiber sheet 11 is preferably higher than a second basis weight of the adhesive 15 applied to the second fiber sheet 12. In this way, due to the different configuration between the application areas of the adhesives 15 and the basis weights of the adhesives 15 on the fiber sheets 11 and 12, the absorbent polymer 13 can be uniformly maintained at an appropriate position on the second fiber sheet 12 having the application area of the adhesive 15 larger than the application area of the adhesive 15 on the first fiber sheet 11 to prevent the absorbent polymer 13 from unintentionally moving or falling off. Further, since the directly joined portion 17 with the second fiber sheet 12 is easily formed via the adhesive 15 applied to the first fiber sheet 11, retentive property of the absorbent polymer 13 between both of the fiber sheets 11 and 12 is further improved. Further, since the application area of the adhesive 15 on the first fiber sheet 11 is smaller than the application area of the adhesive 15 on the second fiber sheet 12, liquid permeability on the first fiber sheet 11 is ensured, whereby a liquid absorption speed can be increased. As a result, liquid absorption performance can be further improved.

The application areas of both the fiber sheets 11 and 12 shall be compared by laminating both the fiber sheets 11 and 12 having the same shape and area such that nonoverlapping portions of the sheets do not occur. Each of the application areas of the adhesive 15 can be calculated in a manner that the existence portions of the adhesives 15 are visualized on the surfaces of the fiber sheets 11 and 12 to which the adhesives 15 are adhered, using ink or toner, and the areas of such portions are calculated using image processing software. When the identification of the existence portions of the adhesives 15 is difficult in the case of using the absorbent sheet 1 as a measurement target, both the fiber sheets 11 and 12 are peeled off using a cold spray, and the above-described method may be applied to the peeled fiber sheets 11 and 12.

In addition, the first basis weight and the second basis weight are measured and calculated using only the applied portions as measurement targets of the fiber sheets 11 and 12 to which the adhesive 15 is applied. Specifically, after the fiber sheets 11 and 12 in the absorbent sheet 1 are separated from each other, a mass A1 (g) of the first fiber sheet 11 is measured to which adhesive 15 is adhered. Further, a total area S ($m^2$) of the existence portions of the adhesives 15 is measured using image processing software in a state where the existence portions of the adhesives 15 are visualized on the surface of the first fiber sheet 11 to which the adhesives 15 are adhered, using ink or toner. Next, the first fiber sheet 11 is immersed in an organic solvent, and a mass A2 (g) of the fiber sheet after the adhered adhesive 15 is dissolved is measured. The first basis weight ($g/m^2$) can be calculated as a formula expressed by "(A1 - A2)/S". Similarly, the second basis weight ($g/m^2$) can be calculated in the same manner as described above for the second fiber sheet 12. Preferred ranges of the first basis weight and the second basis weight will be described below.

From the viewpoint of preventing unintentional movement and falling-off of the absorbent polymer 13 and further increasing the liquid absorption speed, as shown in Figs. 1 and 2, the adhesive 15 is discontinuously applied to the region of the first fiber sheet 11 joined to the second fiber sheet 12 so as to have a non-applied section, and the adhesive 15 is preferably continuously applied to the entire region of the second fiber sheet 12 joined to the first fiber sheet 11 so as not to have a gap. Further, a surface of the first fiber sheet 11 on which the adhesive 15 does not exist, that is, an outer surface of the absorbent sheet 1 on the first fiber sheet 11, is preferably used as a liquid acquisition surface, which is a surface where the absorbent sheet 1 and the liquid first come into contact with each other. The joined region is a region in which the absorbent polymer 13 is disposed in such a manner that no macroscopically recognizable gap is observed.

In the first fiber sheet 11, examples of the form in which the adhesive 15 is discontinuously applied so as to have the non-applied section include a spiral shape, a summit shape, an omega shape, a curtain shape, and a stripe shape. In the embodiment shown in Fig. 2, the adhesive 15 applied to the first fiber sheet 11 has a spiral shape. A preferred method of applying the adhesive 15 to each of the fiber sheets 11 and 12 will be described in detail in a producing method to be described below.

In a description of a positional relation between the absorbent polymer 13 and the adhesive 15 in the thickness direction of the absorbent sheet 1, the region in which the absorbent polymer 13 exists coincides with the region where the adhesive 15 exists.

Specifically, examples of the positional relation include at least one of (a) an aspect where the existence region of the absorbent polymer 13 coincides with the existence region of the adhesive 15 applied to the first fiber sheet 11, but does not coincide with the existence region of the adhesive 15 applied to the second fiber sheet 12, (b) an aspect where existence region of the absorbent polymer 13 coincides with the existence region of the adhesive 15 applied to the second fiber sheet 12, but does not coincide with the existence region of the adhesive 15 applied to the first fiber sheet 11, and (c) an aspect where the existence region of the absorbent polymer 13 coincides with the existence region of the adhesive 15 applied to one fiber sheet, and overlaps with the existence region of the adhesive applied to the other fiber sheet.

In the embodiment shown in Figs. 1 and 2, the existence region of the absorbent polymer 13 coincides with the existence region of the adhesive 15 on the second fiber sheet 12. Further, since the adhesive 15 is discontinuously applied to the first fiber sheet 11 so as to have the non-applied section, there is a portion where the region and the applied region of the adhesive 15 on the first fiber sheet 11 overlap each other.

Returning to the description of the basis weight of the adhesive 15 applied to each of the fiber sheets 11 and 12, a total of the first basis weight and the second basis weight described above is preferably 500 g/m$^2$ or less, more preferably 300 g/m$^2$ or less, and even more preferably 200 g/m$^2$ or less. Further, the total of the first basis weight and the second basis weight is preferably 10 g/m$^2$ or more, more preferably 50 g/m$^2$ or more, and even more preferably 80 g/m$^2$ or more. With such a configuration, since the retentive property between the fiber sheets of the absorbent polymer 13 is enhanced and the swelling of the absorbent polymer 13 is less likely to be inhibited, the liquid absorption performance can be further improved.

From the same viewpoint, the first basis weight is preferably 400 g/m$^2$ or less, more preferably 250 g/m$^2$ or less, even more preferably 100 g/m$^2$ or less, and is practically 20 g/m$^2$ or more. Further, the second basis weight is preferably 30 g/m$^2$ or less, more preferably 15 g/m$^2$ or less, even more preferably 10 g/m$^2$ or less, and is practically 2 g/m$^2$ or more.

In addition, the thickness of the adhesive 15 existing on the surface of the first fiber sheet 11 is preferably thicker than the thickness of the adhesive 15 existing on the surface of the second fiber sheet. With such a configuration, the directly joined portion 17 with the second fiber sheet 12 is easily formed through the adhesive 15 applied to the first fiber sheet 11, and the absorbent polymer 13 can be retained at an appropriate position through the adhesive 15 applied to the second fiber sheet 12, whereby the liquid absorption performance can be further improved.

From the same viewpoint, under a condition of being thicker than the thickness of the adhesive 15 existing on the surface of the second fiber sheet 12, the thickness of the adhesive 15 existing on the surface of the first fiber sheet 11 is preferably 20 $\mu$m or more and more preferably 40 $\mu$m or more, and is preferably 500 $\mu$m or less and more preferably 300 $\mu$m or less. Further, the thickness of the adhesive 15 existing on the surface of the second fiber sheet 12 is preferably 1 $\mu$m or more and more preferably 3 $\mu$m or more, and is preferably 20 $\mu$m or less and more preferably 10 $\mu$m or less. Regarding the thickness of the adhesive 15 existing on the surface of each of the fiber sheets 11 and 12, when the fiber sheet to be measured is cut to a predetermined size, a cross section thereof is observed with the scanning electron microscope (for example, the scanning electron microscope JCM-6000 produced by JEOL Ltd.) at a magnification of 50 to 500 times, the thicknesses of five points of the adhesive 15 protruding from the interface of the fiber sheet to be measured are measured, and an arithmetic average value of the measured thicknesses is used as the thickness of the adhesive 15.

The absorbent sheet 1 has peeling strength between the first fiber sheet 11 and the second fiber sheet 12 in a dry state, the peeling strength being preferably 0.1 N/25 mm or more, more preferably 0.2 N/25 mm or more, and even more preferably 0.3 N/25 mm or more, and being preferably 3 N/25 mm or less, more preferably 2 N/25 mm or less, and even more preferably 1.5 N/25 mm or less. With such peeling strength, sufficient strength can be exhibited at the time of use of the absorbent sheet 1, and strength is maintained even after the liquid absorption of the absorbent sheet. The dry state means that when the absorbent sheet 1 is dried under the condition of 105°C for 8 hours, a mass change of the absorbent sheet 1 is 20 mass% or less before and after drying. The mass change of the absorbent sheet 1 is calculated based on Formula (1) below.

$$\text{Mass change (mass\%)} = 100 \times ([\text{Mass (g) of absorbent sheet 1 before drying}] - [\text{Mass (g) of absorbent sheet 1 after drying}])/([\text{ Mass (g) of absorbent sheet 1 before drying}]) \ldots (1)$$

The peeling strength in the dry state can be measured by the following method, for example. The absorbent sheet being in the dry state is cut out to prepare a test piece. The shape of the test piece is a rectangle having a length in the longitudinal direction of the absorbent sheet exceeding 80 mm and a length in the width direction thereof being 25 mm. At one end of the test piece in the longitudinal direction, the portion between the first fiber sheet 11 and the second fiber sheet 12 is peeled off, and the peeled-off portion is set in each chuck of a tensile tester in a T shape. As the tensile tester, for example, Autograph AG-X produced by Shimadzu Corporation is used. A distance between the chucks is 20 mm. Peeling is performed at a tensile speed of 300 mm/min, and a test force average strength is measured. The measurement is performed three times, and an arithmetic average value of the test force average strength is defined as peeling strength (unit: N/25 mm).

When the absorbent sheet 1 is immersed in physiological saline for 30 minutes, the thickness change thereof is preferably 2 mm or more, more preferably 3 mm or more, even more preferably 4 mm or more, and is practically 20 mm or less. When the absorbent sheet 1 is immersed in the physiological saline for 30 minutes, the thickness change thereof preferably increases within the above range.

In addition, when both the fiber sheets 11 and 12 are peeled off after being immersed in physiological saline for 30 minutes, the number of portions where both the fiber sheets 11 and 12 are directly joined to each other by the adhesive 15 is preferably one or more per 1 cm$^2$, more preferably two or more per 1 cm$^2$, and even more preferably three or more per 1 cm$^2$. With such a configuration, both the swelling property and the fixing property of the absorbent polymer can be improved, and the liquid absorbency is further improved.

The thickness change is measured in such a manner that the absorbent sheet 1 in the dry state is cut out to a size of 5 cm × 5 cm to obtain a test piece in a dry state and a thickness T1 (mm) of the test piece is measured. Next, after the test piece is immersed in physiological saline having an amount of 200 times the mass of the dried test piece for 30 minutes, a thickness T2 (mm) of the test piece is measured. The thickness change (mm) is calculated as a difference between the thickness T2 and the thickness T1. Both the thicknesses T1 and T2 are measured under a load of 1.7 kPa. Further, regarding the portions where both the fiber sheets 11 and 12 are directly joined to each other by the adhesive 15, the number of the portions where the adhesive 15 exists in a columnar shape is measured when the test piece after being immersed in physiological saline is peeled off between both the fiber sheets 11 and 12.

A compression strain ratio of the absorbent sheet 1 in a wet state is preferably 25% or more, more preferably 30% or more, and even more preferably 35% or more, and is practically 70% or less. With such a configuration, since the flexibility of the absorbent sheet 1 can be maintained even after the absorbent polymer swells due to the liquid absorption, the absorbent sheet 1 after the liquid absorption is excellent in tactility. The wet state means a state after the dried absorbent sheet 1 is immersed in physiological saline having an amount of 200 times the mass of the absorbent sheet 1 for 30 minutes, then put on a net and wiped off excess water with paper.

The compression strain ratio can be measured using, for example, a KES-G5 handy compression tester produced by Kato Tech Co., Ltd. Specifically, the absorbent sheet 1 having a size of 5 cm × 5 cm is immersed in physiological saline having an amount of 200 times the mass of the sheet 1 for 30 minutes, and then attached to a test table. Next, the absorbent sheet after immersion is compressed between steel plates having a circular plane with an area of 2 cm$^2$. A compression speed is 20 μm/sec, and a maximum compression load is 50 g/cm$^2$. When a thickness under a load of 49 Pa (0.5 gf/cm$^2$) is defined as a thickness T0 (mm) and a thickness under a load of 4900 Pa (50 gf/cm$^2$) is defined as a thickness Tm (mm), the compression strain ratio (%) can be calculated as "$100 \times (T0 - Tm)/T0$".

Preferably, the absorbent sheet 1 is configured in which the surface of at least one of both the fiber sheets 11 and 12 on which the adhesive 15 does not exist has a projecting-and-depressed structure. In particular, more preferably, the surface of the first fiber sheet 11 on which the adhesive 15 does not exist has a projecting-and-depressed structure, and the surface of the second fiber sheet 12 on which the adhesive 15 does not exist does not have a projecting-and-depressed structure. With such a configuration, a surface area of the fiber sheet can be increased, and a contact area with the liquid can be increased. Further, when the absorbent sheet 1 is used together with other members, friction can be easily generated and slippage can be reduced.

Examples of the projecting-and-depressed structure include a structure where projections and depressions are formed regularly or irregularly in scattered dots, and a shape where ridges and grooves are alternately formed, and preferably the structure where projections and depressions are formed irregularly in scattered dots. Regarding the projecting-and-depressed structure, for example, when a mean deviation of surface roughness SMD measured by the following method is equal to or more than 5 μm, it is defined that "a projecting-and-depressed structure is provided", and when the SMD is less than 5 μm, it is defined that "a projecting-and-depressed structure is not provided".

[Measurement method of mean deviation of surface roughness SMD]

The mean deviation of surface roughness SMD is measured by the following method using KESFB4-AUTO-A (trade name) produced by Kato Tech Co., Ltd. according to a method disclosed in the following book.

Sueo Kawabata, "Standardization and Analysis of Texture Evaluation", 2nd edition, Texture Measurement and Standardization Committee, The Textile Machinery Society of Japan (Jul. 10, 1980).

Specifically, the absorbent sheet 1 is used as it is, or the sheet 1 is cut out to prepare a test piece of 10 cm × 10 cm. The test piece is placed on a test table having a smooth plane metal surface. Next, a piano wire having a diameter of 0.5 mm bent into a U shape with a width 5 mm is used as a contactor, and a contact surface of the contactor is crimped against the test piece with a force 9.8 cN (within an error ± 0.49 cN). In this state, the test piece is moved horizontally by 2 cm at a constant speed of 0.1 cm/sec. A uniaxial tension of 19.6 cN/cm is applied to the test piece. The contactor is in a state of being crimped by a spring, a spring constant is set to 24.5 cN/mm (within an error ± 0.98 cN/mm), and a resonance frequency is set to 30 Hz or more in a state of being away from the surface contact.

A measured value of the mean deviation of surface roughness is expressed as an SMD value. The measurement is performed in a direction (MD direction) along one side of the test piece and a direction (CD direction) orthogonal to the MD direction, and an SMD value ($SMD_{MD}$) in the MD direction and an SMD value ($SMD_{CD}$) in the CD direction are obtained. From the obtained $SMD_{MD}$ and $SMD_{CD}$, the mean deviation of surface roughness SMD (μm) is calculated from Formula (2) below. When the test piece of 10 cm × 10 cm cannot be prepared, it is calculated using the SMD value in either the MD direction or the CD direction.

$$\text{Mean deviation of surface roughness SMD } (\mu\text{m}) = \{(\text{SMD}_{MD}^2 + \text{SMD}_{CD}^2)/2\}^{1/2} \dots (2)$$

In a description of the absorbent polymer 13 included in the absorbent sheet 1, the basis weight of the absorbent polymer 13 is preferably 60 g/m$^2$ or more, more preferably 80 g/m$^2$ or more, and even more preferably 100 g/m$^2$ or more, and is preferably 700 g/m$^2$ or less, more preferably 500 g/m$^2$ or less, and even more preferably 400 g/m$^2$ or less. When the basis weight of the absorbent polymer 13 is within such a range, it is possible to efficiently manufacture the absorbent sheet 1 having both of the liquid absorbency of the absorbent polymer 13 and the retentive property of the absorbent polymer 13 due to the directly joined portion 17.

From the same viewpoint, the median particle size of the absorbent polymer 13 is preferably 800 $\mu$m or less, more preferably 650 $\mu$m or less, and even more preferably 500 $\mu$m or less. Further, from the viewpoint of improving handling at the time of producing the absorbent sheet, the median particle size of the absorbent polymer 13 is preferably 10 $\mu$m or more, more preferably 100 $\mu$m or more, and even more preferably 150 $\mu$m or more. The median particle size of the absorbent polymer 13 can be obtained as a particle size corresponding to a cumulative ratio of 50% in particle size distributions that are obtained using a laser diffraction/scattering type particle size distribution measuring device (LA-920, produced by HORIBA, Ltd.) and are graphed as a cumulative distribution. Measurement conditions are as follows: add the absorbent polymer 13 to a dispersion medium in which 90 mass% of ethanol of and 10 mass% of distilled water are mixed so as to be 0.1 mass%, and perform dispersion treatment on the mixture with stirring and built-in ultrasonic waves for 3 minutes. The polymer dispersion solution subjected to the dispersion treatment is measured by a flow method with the above-described measuring device to obtain a median particle size.

The absorbent polymer 13 included in the absorbent sheet 1 has a swelling rate of preferably 80% or more, more preferably 85% or more, and even more preferably 90% or more. Additionally, the peeling strength between the first fiber sheet 11 and the second fiber sheet 12 in the wet state is preferably 0.1 N/25 mm or more, more preferably 0.2 N/25 mm or more, and even more preferably 0.3 N/25 mm or more, and is preferably 2.0 N/25 mm or less, more preferably 1.5 N/25 mm or less, and even more preferably 1.0 N/25 mm or less. By such swelling rate and peeling strength, the liquid absorbency of the absorbent polymer and the sheet strength after liquid absorption become further excellent.

The swelling rate of the absorbent polymer can be measured by the following method, for example. First, a nylon woven fabric (sold by Sanriki Seisakusyo Co., Ltd., product name: nylon net, standard: 250 mesh) is cut into a rectangle having a width of 15 cm and a length of 40 cm to produce a nylon bag having a width of 15 cm (internal size of 14 cm) and a length of 20 cm by folding it in half at a center in a longitudinal direction and heat-sealing at both ends thereof.

Next, two dried absorbent sheets 1 are prepared, and the same portion of the sheets 1 is cut out to a size of 10 cm $\times$ 10 cm to obtain test pieces. One of the test pieces is housed in the nylon bag without being bent and is immersed in physiological saline having an amount of 200 times the mass of the test piece for 30 minutes, and then the test piece is dehydrated by centrifugal treatment with 143$\times$g for 10 minutes in a state of being housed in the nylon bag. A mass X1 (g) of the dehydrated test piece contained in the nylon bag and a mass Y1 (g) of only the dehydrated nylon bag are measured.

Separately, the other test piece (dry state) is separated into a first fiber sheet, a second fiber sheet, and an absorbent polymer by using a cold spray, and all of these separated members are housed in one nylon bag without bending the respective fiber sheets. Then, the test piece is immersed in physiological saline having an amount of 200 times the mass of the test piece for 30 minutes, and then the test piece dehydrated by centrifugal treatment with 143$\times$g for 10 minutes in a state of being housed in the nylon bag. A mass X2 (g) of the dehydrated test piece contained in the nylon bag and a mass Y2 (g) of only the dehydrated nylon bag are measured.

The swelling rate (%) is calculated based on a formula represented by "$\{(X1 - Y1)/(X2 - Y2)\} \times 100$". The peeling strength between both the fiber sheets 11 and 12 in the wet state can be measured in the same manner as the peeling strength in the dry state after the absorbent sheet to be measured is impregnated with physiological saline under the above-described conditions to make it in a wet state.

For a distribution of the absorbent polymers 13 existing in the absorbent sheet 1, the absorbent polymer 13 preferably exist uniformly in the surface direction of the sheet 1 from the viewpoint of reducing the formation of unintended projections and depressions on the entire absorbent sheet 1 after the swelling of the absorbent polymers 13. The uniform existence means that the change in the basis weight of the absorbent polymer 13 measured at any portion of the absorbent sheet is 0.5 or less as a coefficient of variation represented by a formula of standard deviation of basis weights/arithmetic average value of basis weights.

On the other hand, from the viewpoint of further improving the liquid absorption performance at the central region of the sheet while maintaining the liquid absorbency and the retentive property of the absorbent polymer and preventing the absorbent polymer from falling off from the end of the sheet, the amount of the absorbent polymers existing in the central region of the absorbent sheet 1 is preferably larger than the amount of the absorbent polymers 13 existing at both ends in one direction of the absorbent sheet 1, and the amount of the absorbent polymers existing in the central region of the absorbent sheet 1 is more preferably larger than the amount of the absorbent polymers 13 existing in a peripheral region of the absorbent sheet 1.

When the amount of the absorbent polymers existing in the central region of the absorbent sheet 1 is larger than the amount of the absorbent polymers 13 existing at both ends in one direction of the absorbent sheet 1 or the amount of the absorbent polymers 13 existing in the peripheral edge, the number of directly joined portions 17 existing at both ends in one direction or at the peripheral edge of the absorbent sheet 1 is preferably larger than the number of directly joined portions 17 in the central region of the absorbent sheet 1. In this case, an application pattern of the adhesive in the central region of the absorbent sheet 1 is preferably equal to an application pattern of the adhesive at both ends in one direction or the peripheral edge of the absorbent sheet 1. With such a configuration, since the adhesive can be applied to each of the fiber sheets in the same pattern at the time of producing the absorbent sheet 1, workability can be improved and the falling-off of the absorbent polymer from the end of the sheet can be prevented due to improvement of an adhesive force at a periphery of the sheet.

The shape of the absorbent polymer 13 used in the present embodiment is not particularly limited, and may be a spherical particle shape, a tufted particle shape, a lump particle shape, a barrel particle shape, a fibrous particle shape, an undefined particle shape, and a combination particle shape thereof. From the viewpoint of improving the uniformity of spraying of the absorbent polymer 13 at the time of producing the absorbent sheet 1 and easily forming the absorbent sheet 1 including the directly joined portion 17, the absorbent polymer 13 preferably the same particle, and the coefficient of variation of particles of the absorbent polymer 13 is more preferably 0.5 or less in the particle size distribution.

The coefficient of variation in the particle size distribution can be measured by the following method, for example. That is, using a laser diffraction/scattering type particle size distribution measuring device (LA-920, produced by HORIBA, Ltd.), the absorbent polymer 13 is added to a dispersion medium in which 90 mass% of ethanol of and 10 mass% of distilled water are mixed so as to be 0.1 mass%, and the mixture is subjected to dispersion treatment with stirring and built-in ultrasonic waves for 3 minutes. The polymer dispersion solution subjected to the dispersion treatment is measured by the flow method with the above-described measuring device to obtain an arithmetic average value of the particle size and a standard deviation of the particle size. A value calculated by the resulting standard deviation/arithmetic average value is used as the coefficient of variation.

Further, a liquid absorption amount (hereinafter, also referred to as "absorption amount under pressure") of the absorbent polymer 13 is preferably 15 g/g or more, more preferably 20 g/g or more, and even more preferably 25 g/g or more in a state where a pressure of 2.0 kPa is applied. By using such an absorbent polymer, since the absorbent polymer 13 can sufficiently absorb the liquid and swell even when the absorbent polymer 13 is pressed between both the fiber sheets 11 and 12, both the liquid absorbency and the supportability of the absorbent polymer are excellent. The absorbent polymer having the above-described absorption amount under pressure can be obtained by appropriately changing the material, particle size, degree of cross-linking of the absorbent polymer, for example.

The absorption amount under pressure can be measured according to a method disclosed in Japanese Patent Laid-Open No. 2019-34229. That is, a column having a mesh (250 mesh) attached to a lower end opening of a vertically standing cylinder having an inner diameter of 30 mm is prepared, and 0.5 g of absorbent polymer particles are put into the column so as to have a uniform thickness. Next, a weight having a size slightly smaller than an outer diameter of 30 mm is placed on the absorbent polymer so as to load a pressure of 2.0 kPa to the absorbent polymer. Separately, a 100 mL beaker containing 100 mL of physiological saline at room temperature (20 ± 5°C) is prepared. Subsequently, the column containing the absorbent polymer and the weight is immersed in the physiological saline for one hour such that the mesh of the column does not abut on the bottom of the beaker. Then, the column is taken out from the beaker, and is allowed to stand for 15 minutes in the state where the weight is placed on the absorbent polymer, and the column is dehydrated at room temperature (20 ± 5°C). The absorption amount under pressure (g/g) is calculated based on Formula (3) below.

[Math. 1]

$$\text{Absorbent amount under pressure (g/g)} = \frac{(\text{Total mass}) - (\text{Mass of column}) - (\text{Mass of absorbent polymer}) - (\text{Mass of weight})}{(\text{Mass of absorbent polymer})} \quad (3)$$

As the absorbent polymer 13, a polymer or copolymer of acrylic acid or an alkali metal salt of acrylic acid can generally be used. Examples thereof include polyacrylic acid and salts thereof, and polymethacrylic acid and salts thereof, and specifically include sodium salt in acrylic acid polymerization.

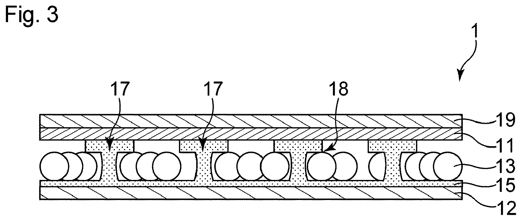

Fig. 3 shows an absorbent sheet according to another embodiment of the present invention. As shown in Fig. 3, the absorbent sheet 1 preferably further includes a fiber-entangling layer 19 disposed adjacent to at least one fiber sheet of both the fiber sheets 11 and 12, and a surface disposed with the fiber-entangling layer 19 is preferably a liquid acquisition surface. In the embodiment shown in Fig. 3, the fiber-entangling layer 19 is disposed adjacent to the first fiber sheet 11 and is not adjacent to the second fiber sheet 12. With such a configuration, it is possible to reduce a change in unevenness of the sheet due to the swelling of the absorbent polymer after the liquid absorption and to improve adhesion between the fiber-entangling layer and the fiber sheet, so that the liquid can easily be drawn into the absorbent polymer and the liquid absorbency becomes further excellent.

The fiber-entangling layer 19 has an aspect of the above-described fiber sheet, or has a bulky structure in which a thickness is thicker than the thickness of the fiber sheet and constituting materials are stacked. Examples of the materials constituting the fiber-entangling layer 19 include various fibers used for the fiber sheet and the above-described absorbent polymer. Examples of the form of the fiber-entangling layer 19 include fiber sheets such as paper, woven fabric, and nonwoven fabric, or a mixed stacked fiber body of the fiber such as pulp and the absorbent polymer.

From the viewpoint of further enhancing a liquid draw property, at least one of the first fiber sheet and the second sheet adjacent to the fiber-entangling layer 19 preferably has a Klemm water absorption height of 10 mm or more, more preferably 20 mm or more, and even more preferably 30 mm or more, and preferably uses a nonwoven fabric or paper including a hydrophilic fiber in particular. Particularly, as will be described below, when paper having a crepe (crease) is used as the fiber sheet adjacent to the fiber-entangling layer, the crepe can increase a capillary force to increase the Klemm water absorption height, rigidity of the fiber sheet can be reduced after the liquid is retained to increase the adhesion with the fiber-entangling layer, and the liquid draw property toward the absorbent polymer can be further enhanced. The Klemm water absorption height is a liquid-retentive index, and the higher the Klemm water absorption height, the higher the liquid-retentive property of the fiber sheet. The Klemm water absorption height can be measured according to JIS P8141 by, for example, a method to be described below. Further, the crepe will be described below.

The first fiber sheet 11 and the second fiber sheet 12 are preferably configured such that the Klemm water absorption heights are different. Specifically, when the Klemm water absorption height is measured based on a measurement method to be described below, the Klemm water absorption height of the first fiber sheet 11 is higher than the Klemm water absorption height of the second fiber sheet 12. Since each of the fiber sheets has such a physical property, on the first fiber sheet 11, the liquid can be easily retained in the fiber sheet 11 to improve a liquid diffusive property in the surface direction of the sheet, and the diffused liquid is brought into contact with the respective absorbent polymers 13, whereby the utilization efficiency of the respective absorbent polymers 13 can be improved. As a result, the liquid absorbency can be enhanced. Additionally, on the second fiber sheet 12, since the liquid-retentive property of the second fiber sheet 12 itself is lower than that of the first fiber sheet 11, an air-permeability is improved.

From the viewpoint of making the above-described effects remarkable, a Klemm water absorption height C1 of the first fiber sheet 11 is preferably 10 mm or more, more preferably 20 mm or more, even more preferably 25 mm or more, and further still more preferably 30 mm, and is practically 60 mm or less.

From the same viewpoint, a Klemm water absorption height C2 of the second fiber sheet 12 is preferably 30 mm or less, more preferably 20 mm or less, and even more preferably 10 mm or less, and is practically 1 mm or more, under a condition of C1 > C2.

From the same viewpoint, an outer surface of the absorbent sheet 1 on the first fiber sheet 11 is preferably also used as a water acquisition surface which is a surface where the absorbent sheet 1 and the liquid firstly come into contact with each other. With such a configuration, the utilization efficiency of the absorbent polymer 13 can be improved as compared with a case where an outer surface on the second fiber sheet 12 is used, so that there is an advantage that the liquid absorbency is excellent. The Klemm water absorption heights C1 and C2 of the respective fiber sheets 11 and 12 can be appropriately adjusted by changing the basis weight and the thickness of the fiber sheet or the material of the fibers constituting the fiber sheets.

From the same viewpoint, a difference (C1 - C2) between the Klemm water absorption height C1 of the first fiber sheet 11 and the Klemm water absorption height C2 of the second fiber sheet 12 is, under the condition of C1 > C2, preferably 10 mm or more, more preferably 15 mm or more, and even more preferably 20 mm or more, and is preferably 50 mm or less, more preferably 45 mm or less, and even more preferably 40 mm or less.

The Klemm water absorption height can be measured by the following method according to a test method of JIS P8141, for example. Specifically, the fiber sheet to be measured is taken out from the absorbent sheet 1 while being careful not to change the thickness of the fiber sheet. When the fiber sheet to be measured is joined to another member with an adhesive, the adhesive is solidified by a cooling means such as cold spraying, and then the fiber sheet is taken out. Further, when the absorbent polymer 13 is adhered to the fiber sheet to be measured, similarly, the adhesive is solidified using cold spraying, and then the absorbent polymer 13 is removed. The fiber sheet to be measured is cut out to a size of a width of 30 mm and a length of 100 mm or more to obtain a sample.

When the absorbent sheet has a rectangular shape that is long in one direction in a plan view, the fiber sheet to be measured is cut such that the longitudinal direction of the absorbent sheet matches the longitudinal direction of the sample. When the absorbent sheet has a square shape in a plan view, the fiber sheet to be measured is cut to obtain a sample such that a direction along any one side matches the longitudinal direction of the sample. Further, when the absorbent sheet is a non-polygonal shape such as a circle in a plan view, in consideration of a virtual circle centered on a centroid of the absorbent sheet 1 in a plan view and a virtual radial line that passes through the center of the virtual circle and cuts the virtual circle at intervals of 30°, the fiber sheet to be measured is cut at intervals 30° with the virtual radial line to obtain a sample such that an extending direction of the virtual radial line matches a longitudinal direction of the sample. Further, when the absorbent sheet 1 is incorporated into the absorbent article, the fiber sheet to be measured is taken out from the absorbent article and the absorbent sheet 1 according to the above-described method, and is cut out to the above-described size such that the longitudinal direction of the absorbent article matches the longitudinal direction of the sample.

During cutting regardless of the shape of the absorbent sheet in a plan view or whether the absorbent sheet is incorporated into the absorbent article, cutting methods using a push cutter may be likely to affect results because of crushing the sample, which is not preferable. For this reason, cutting is performed using a knife, a cutter, and a razor so as not crush a cut surface of the sample. Using each cut sample, the Klemm water absorption height (mm) after 5 minutes is measured according to JIS P8141. The above measurement is performed 10 times for each sample, and an arithmetic average value thereof is taken as the Klemm water absorption height (mm) of the sample.

From the viewpoint of further improving the liquid absorbency and exhibiting the excellent air-permeability at the same time by facilitating vapor generated by evaporation of the absorbed liquid to escape to the outside through the second fiber sheet 12, an air-permeability resistance R2 of the second fiber sheet 12 is preferably lower than an air-permeability resistance R1 of the first fiber sheet 11.

Specifically, the air-permeability resistance R1 of the first fiber sheet 11 is preferably 0.1 kPa·s/m or more, more preferably 0.2 kPa·s/m or more, and even more preferably 0.3 kPa·s/m or more, and is preferably 0.8 kPa·s/m or less, more preferably 0.6 kPa·s/m or less, and even more preferably 0.4 kPa·s/m or less.

Further, the air-permeability resistance R2 of the second fiber sheet 12 is, under a condition of being lower than the air-permeability resistance R1 of the first fiber sheet 11, preferably 0.005 kPa·s/m or more, more preferably 0.01 kPa·s/m or more, and even more preferably 0.02 kPa·s/m or more, and is preferably 0.3 kPa·s/m or less, more preferably 0.2 kPa·s/m or less, and even more preferably 0.1 kPa·s/m or less.

The lower the above-described air-permeability resistance, the higher the air-permeability, and the air-permeability resistance can be measured by a KES-F8 air-permeability tester (AUTOMATIC AIR-PERMEABILITY TESTER KES-F8-AP1 produced by Kato Tech Co., Ltd.).

From the same viewpoint, a difference (R1 - R2) between the air-permeability resistance R1 of the first fiber sheet 11 and the air-permeability resistance R2 of the second fiber sheet 12 is, under a condition of R1 > R2, preferably 0.07 kPa·s/m or more, more preferably 0.15 kPa·s/m or more, and even more preferably 0.2 kPa·s/m or more, and is preferably 0.7 kPa·s/m or less, more preferably 0.5 kPa·s/m or less, and even more preferably 0.4 kPa·s/m or less.

From the viewpoint of facilitating the diffusion of the liquid in the surface direction of the sheet, further improving the utilization efficiency of the absorbent polymer 13, and achieving higher liquid absorbency, the first fiber sheet 11 is preferably paper. The paper is produced by agglutinating plant fibers such as pulp or other fibers, preferably in a wet manner, according to the provisions of JIS P0001.

When the first fiber sheet 11 is paper, a crepe (a fold) is preferably provided, and a crepe ratio F1 of the first fiber sheet 11 is preferably 5% or more, more preferably 10% or more, and even more preferably 15% or more, and is practically 30% or less. Further, since the capillary force of the fiber sheet can be increased by such a crepe ratio, the fiber sheet having the above-described Klemm water absorption height can be easily obtained. Such a fiber sheet may be subjected to a known crepe treatment so as to have the above-described crepe ratio.

The crepe ratio can be measured by an underwater elongation method, for example, based on the following method. Measurement is performed at 23 ± 2°C and relative humidity of 50 ± 5%, and a sample is stored in the same environment for 24 hours or longer prior to the measurement and then is measured. The fiber sheet to be measured is cut to a size of 25 mm in a direction in which the creases extend and a size of 100 mm in a direction orthogonal to the extending direction to prepare a measurement sample, the measurement sample is immersed in water for one minute and then pulled up, and the crepe ratio is calculated by Formula (4) below from the amount of change in the size in the orthogonal direction. The measurement is performed three times, and an arithmetic average value of the measured values is taken as a crepe ratio (%). When the size of 100 mm cannot be secured in the orthogonal direction, the crepe ratio can be obtained by cutting the fiber sheet to a size of at least 30 mm or more in the orthogonal direction.

$$\text{Crepe ratio (\%)} = ((\text{Size after immersion in water (mm)})/(\text{Size before immersion in water (mm)}) - 1) \times 100 \quad \dots(4)$$

Regarding the second fiber sheet 12, the fiber sheet 12 preferably has a crepe ratio F2 of less than 1% which is measured according to the above-described method. Since the second fiber sheet 12 has such a crepe ratio, the liquid is hardly retained in the second fiber sheet 12, whereby it is possible to obtain the sheet having higher air-permeability, and to easily obtain the fiber sheet having the Klemm water absorption height lower than that of the first fiber sheet 11.

When the first fiber sheet 11 is paper, the fiber of the paper preferably has an orientation propensity in one direction. With such a configuration, since it is possible to form a direction in which the Klemm water absorption height is high and a direction in which the Klemm water absorption height is low, respectively, with a single fiber sheet, the liquid diffusive property can be enhanced in a direction having the orientation propensity, the utilization efficiency of the absorbent polymer can be improved, and the liquid can be prevented from being unintentionally diffused in a direction orthogonal to the direction having the orientation propensity. In particular, as will be described below, when the absorbent sheet 1 having an orientation propensity in one direction is incorporated such that the direction having the orientation propensity matches the longitudinal direction of the absorbent article, the liquid diffusive property can be enhanced in the longitudinal direction of the absorbent article, the utilization efficiency of the absorbent polymer can be improved, and liquid leakage in the width direction can be prevented by inhibiting the unintended diffusion in the width direction of the absorbent article.

The orientation propensity is a value measured according to a measurement method to be described below, and is preferably 60% or more and more preferably 70% or more.

The orientation propensity of the fibers constituting the fiber sheet is measured using a tensile compression tester (AG-IS produced by Shimadzu Corporation). A test piece is cut out with a length of 150 mm and a width of 50 mm in one direction of the fiber sheet and in a direction orthogonal thereto. When the fiber sheet has a square or rectangular shape, the test piece may be cut out so as to have the above-described size with a direction along any one side as a longitudinal direction and a direction orthogonal to the longitudinal direction as a width direction. For the test piece, a distance between chucks is 100 mm, the test piece is pulled at a tensile speed of 300 mm/min to extend in the longitudinal direction, and the maximum load (N) at fracture is recorded. This experiment is performed on five test pieces, the maximum loads of the respective test pieces are measured, and an arithmetic average value of the measured maximum loads is taken as a tensile strength (N) in the longitudinal direction. Similarly, the five test pieces having the above-described size are pulled to extend in the width direction, the maximum loads (N) at fracture of the respective test pieces are measured, and an arithmetic average value of the measured maximum loads is taken as a tensile strength (N) in the width direction.

Based on the tensile strength in the longitudinal direction and the tensile strength in the width direction obtained by the above-described method, the orientation propensity is calculated from Formula (5) below. When the orientation propensity calculated from Formula (5) below is 50% or more, it is assumed that "the orientation propensity is in the direction along the longitudinal direction of the test piece", and when the orientation propensity calculated from Formula (5) below is less than 50%, it is assumed that "the orientation propensity is in the width direction of the test piece". The longitudinal direction and the width direction of the test piece correspond to one direction and the orthogonal direction thereof in the cut fiber sheet, respectively.

$$\text{Orientation propensity (\%)} = 100 \times (\text{[Tensile strength in longitudinal direction (N)]}/(\text{[Tensile strength in longitudinal direction (N)]} + \text{[Tensile strength in width direction (N)]})) \dots(5)$$

In particular, from the viewpoint of further making the liquid absorbency remarkable, the first fiber sheet 11 is preferably paper, the second fiber sheet 12 is preferably nonwoven fabric, and as shown in Fig. 3, the fiber-entangling layer 19 is preferably disposed adjacent to the first fiber sheet 11. In this case, the fiber-entangling layer 19 may be a fiber layer containing a mixture of hydrophilic pulp and an absorbent polymer. The fiber-entangling layer 19 can be, for example, an absorbent member to be described below.

From the viewpoint of further improving the liquid absorbency, the basis weight of the first fiber sheet 11 is preferably 6 g/m$^2$ or more and more preferably 8 g/m$^2$ or more, and is preferably 50 g/m$^2$ or less, more preferably 30 g/m$^2$ or less, and even more preferably 20 g/m$^2$ or less.

From the same viewpoint, the basis weight of the second fiber sheet 12 is preferably 4 g/m$^2$ or more and more preferably 6 g/m$^2$ or more, and is preferably 30 g/m$^2$ or less and more preferably 20 g/m$^2$ or less.

From the same viewpoint, the thickness of the first fiber sheet 11 is preferably 0.04 mm or more and more preferably 0.08 mm or more, and is preferably 1 mm or less and more preferably 0.3 mm or less.

From the same viewpoint, the thickness of the second fiber sheet 12 is preferably 0.03 mm or more and more preferably 0.07 mm or more, and is preferably 0.5 mm or less and more preferably 0.2 mm or less. The thickness of each of the fiber sheets 11 and 12 is measured by a laser displacement meter under a pressure of 1.7 kPa.

The thickness of the absorbent sheet is preferably 0.3 mm or more, and more preferably 0.6 mm or more from the viewpoint of improving the liquid absorbency. Further, the thickness of the absorbent sheet is preferably 4 mm or less, more preferably 3 mm or less, and even more preferably 2 mm or less from the viewpoint of improving the usability of the user when the absorbent sheet or the absorbent article containing the sheet is used. The thickness of the absorbent sheet described above is the thickness of the entire absorbent sheet measured under a pressure of 1.7 kPa.

The above-described absorbent sheet 1 may be used as it is. In this case, the absorbent sheet 1 may be used as a single sheet, or a plurality of absorbent sheets may be used in a laminated state.

Further, the above-described absorbent sheet 1 can be used as a constituent member of the absorbent article. Typically, the absorbent article has a longitudinal direction along a front-rear direction of a wearer and a width direction orthogonal to the longitudinal direction, and includes a topsheet and a backsheet, and the absorbent article can be used in a state where the absorbent sheet of the present invention is disposed between the topsheet and the backsheet. Examples of the absorbent article include an incontinence pad, a sanitary napkin, and a disposable diaper. From the viewpoint of improving the liquid absorbency of the absorbent article, when the absorbent sheet 1 is used as a constituent member of the absorbent article, a plurality of absorbent sheets 1 may be used in a laminated state, and an absorbent member may be further used in a state of being laminated on the absorbent sheet 1. When the absorbent member is used in the state of being laminated on the absorbent sheet 1, the absorbent member is preferably disposed at least in the central region of the absorbent sheet 1.

The topsheet used for the absorbent article is a sheet constituting a surface (hereinafter, also referred to as a "skin-facing surface") facing the skin of the wearer who wears the absorbent article when the absorbent article is worn at an appropriate position, and the backsheet is a sheet constituting a surface (hereinafter, also referred to as a "non-skin-facing surface") facing the side opposite to the skin of the wearer who wears the absorbent article. As the topsheet and the backsheet used for the absorbent article, the topsheet and the backsheet conventionally used for the absorbent article can be used without particular limitation. For example, various liquid-permeable nonwoven fabrics or perforated films can be used as the topsheet. As the backsheet, a sparingly liquid permeable or water repellent resin film or a laminate of a resin film and a nonwoven fabric can be used.

The absorbent member used for the absorbent article includes an absorbent core. The absorbent core includes, for example, a stacked fiber body of hydrophilic fibers such as cellulose including pulp, a mixed stacked fiber body of the hydrophilic fiber and the absorbent polymer, and a deposit body of the absorbent polymer. At least the skin-facing surface of the absorbent core may be covered with a liquid-permeable core-wrap sheet, and the entire surface including the skin-facing surface and the non-skin-facing surface may be covered with a core-wrap sheet. As the core-wrap sheet, for example, thin paper made of hydrophilic fibers or a liquid-permeable nonwoven fabric can be used.

In particular, when the absorbent sheet 1 is used as a constituent member of the absorbent article, the first fiber sheet 11 of the absorbent sheet 1 is preferably disposed so as to be a water acquisition surface. In other words, the absorbent sheet 1 is preferably disposed such that the first fiber sheet 11 is disposed closer to the skin-facing surface. Further, when the first fiber sheet 11 is subjected to creping, the absorbent sheet is also preferably disposed such that the extending direction of crepes matches the longitudinal direction of the absorbent article. Further, when the fibers constituting the first fiber sheet 11 or both the fiber sheets 11 and 12 has an orientation propensity in one direction, the absorbent sheet is also preferably disposed such that a direction having the orientation propensity matches the longitudinal direction of the absorbent article. When the absorbent sheet is disposed in this way, both of the liquid draw property from the topsheet and the liquid-retentive property on the absorbent sheet are enhanced, and the absorbent article having high liquid absorbency is obtained. Further, since the liquid adhering to and permeating the topsheet can be easily drawn into the absorbent sheet side to improve dry feeling of the topsheet, there is also an advantage that the feeling of use of the wearer of the absorbent article is improved. Further, since vapor derived from the absorbed liquid can be easily evaporated from the second fiber sheet 12 to reduce the stuffiness when the absorbent article is worn, there is also an advantage that the feeling of use of the wearer of the absorbent article is improved. Evaporation of vapor from the second fiber sheet 12 is particularly effective when a moisture permeable material is used as the backsheet of the absorbent article.

In particular, when the first fiber sheet 11 is used for the absorbent article so as to be disposed closer to the skin-facing surface, the surface of the first fiber sheet 11 on which the adhesive 15 does not exist preferably has the projecting-and-depressed structure described above. With such a configuration, a friction force with other constituent members of the absorbent article can be increased, slippage between the constituent members can be reduced, and the contact area with the skin side of the wearer can be reduced, whereby the feeling of use of the wearer of the absorbent article is improved. Further, when the first fiber sheet 11 is used for the absorbent article so as to be disposed closer to the skin-facing surface, the surface of the second fiber sheet 12 on which the adhesive 15 does not exist disposed closer to the non-skin-facing surface does not preferably have the projecting-and-depressed structure described above. With such a configuration, the outer surface of the absorbent article can be smoothed, and thus the tactility of the absorbent article is improved.

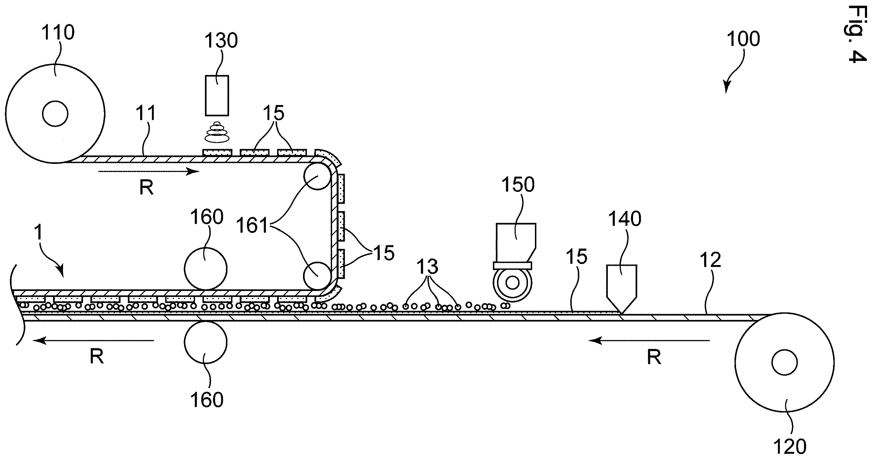

The absorbent sheet of the present invention and the absorbent article including the sheet are described above, and hereinafter, a suitable producing method of the absorbent sheet of the present invention will be described below. Fig. 4 shows an embodiment of a producing apparatus 100 suitably used in the producing method.

The producing apparatus 100 shown in Fig. 4 includes a first raw fabric roller 110 and a second raw fabric roller 120. The first raw fabric roller 110 can feed a long continuous first fiber sheet 11 in a conveyance direction R and supply it to a first adhesive applying unit 130 to be described below. The second raw fabric roller 120 can feed a long continuous second fiber sheet 12 in a conveyance direction R and supply it to a second adhesive applying unit 140 to be described below.

The producing apparatus 100 includes a first adhesive applying unit 130 and a second adhesive applying unit 140. The first adhesive applying unit 130 can continuously or intermittently apply adhesive 15 to one surface of the first fiber sheet 11 supplied from the first raw fabric roller 110. The first adhesive applying unit 130 shown in Fig. 4 is configured to intermittently apply the adhesive 15. The second adhesive applying unit 140 can continuously or intermittently apply adhesive 15 to one surface of the second fiber sheet 12 supplied from the second raw fabric roller 120. The second adhesive applying unit 140 shown in Fig. 4 is configured to continuously apply the adhesive 15. Fig. 4 shows a state in which the adhesive 15 applied to the first fiber sheet 11 and the adhesive 15 applied to the second fiber sheet 12 are applied using the same type of adhesive, but may be applied with different types of adhesives.

The producing apparatus 100 includes a polymer spraying unit 150. The polymer spraying unit 150 is located above the second fiber sheet 12, and can spray an absorbent polymer 13 on the surface of the second fiber sheet 12 applied with the adhesive 15.

The producing apparatus 100 preferably further includes a press roller 160. The press roller 160 presses a laminated body in which the first fiber sheet 11 is superposed on the second fiber sheet 12 containing the absorbent polymer 13 through a guide roller 161. Thus, a long continuous absorbent sheet 1 can be formed in which many directly joined portions 17 are formed.

The producing apparatus 100 may include a sheet cutting unit (not shown) downstream of the press roller 160. The sheet cutting unit cuts the long continuous absorbent sheet 1 at a predetermined position to form an absorbent sheet 1 having a predetermined size. An example of the sheet cutting unit may include a cutter roller having a cutter blade extending in a direction orthogonal to the conveyance direction R.

The producing method of the absorbent sheet using the producing apparatus 100 having the above-described configuration is as follows. First, the adhesive 15 is applied onto at least one of the fiber sheets 11 and 12, preferably, onto each of one surface of the first fiber sheet 11 and one surface of the second fiber sheet 12. In Fig. 4, the adhesive 15 supplied from the first adhesive applying unit 130 is applied onto one surface of the first fiber sheet 11. Further, the adhesive 15 supplied from the second adhesive applying unit 140 is applied onto one surface of the second fiber sheet 12. The adhesive 15 may be applied onto one fiber sheet and then onto the other fiber sheet in sequence, or may be applied onto the respective fiber sheets 11 and 12 at the same time. When the adhesive 15 is applied only onto one fiber sheet of the fiber sheets 11 and 12, the adhesive 15 supplied from either the first adhesive applying unit 130 or the second adhesive applying unit 140 may be applied.

From the viewpoint of efficiently forming the directly joined portion 17 on the absorbent sheet 1 by bonding the adhesives applied onto the respective fiber sheets 11 and 12, an applying method in the first adhesive applying unit 130 is preferably a method in which the basis weight of the adhesive 15 in the applied portion of the adhesive 15 can be adjusted so as to be increased, and more specifically, it is preferable to adopt a pattern applying method, for example, a spiral application, a summit application or an omega application by which a non-applied section of the adhesive 15 is formed. From the same viewpoint, an applying method in the second adhesive applying unit 140 is a preferably a method in which the adhesive 15 can be continuously applied with a low basis weight, more preferably a spray application or a coater application, and particularly preferably a coater application. The basis weight applied to each of the fiber sheets 11 and 12 may be adjusted so as to be within the above-described range.

Next, the polymer spraying unit 150 sprays the absorbent polymer 13 onto the application surface of the adhesive 15 on the second fiber sheet 12. The absorbent polymer 13 is supported in a manner of adhering to the adhesive 15 on the second fiber sheet 12. Preferably, the absorbent polymer 13 is uniformly sprayed in the surface direction of the second fiber sheet 12. In the spraying of the absorbent polymer 13, the spraying amount of the absorbent polymer 13 in the surface direction of the second fiber sheet 12 may be changed according to the intended absorbent sheet 1.

Subsequently, the respective fiber sheets 11 and 12 are superposed such that the application surfaces of the adhesives on the respective fiber sheets 11 and 12 face each other. The laminated body obtained by superposing the respective fiber sheets 11 and 12 is that the absorbent polymer 13 is disposed between the respective fiber sheets 11 and 12. The laminated body may be directly supplied to a sheet cutting unit without being introduced into the press roller 160 to obtain the absorbent sheet of the present invention as necessary. Alternatively, both the fiber sheets 11 and 12 may be directly introduced into the press roller 160 and superposed while being pressed without being superposed in advance.

Subsequently, the superposed both the fiber sheets 11 and 12 are introduced between the press rollers 160 and pressed. Thus, the adhesives 15 applied onto the respective fiber sheets 11 and 12 can be easily bonded directly, and thus the directly joined portion 17 can be easily formed. Additionally, the adhesiveness between the absorbent polymer 13 and the adhesive 15 can be enhanced. As a result, both the liquid absorbency and the retentive property of the absorbent polymer can be improved at the same time.

From the viewpoint of efficiently forming the directly joined portion 17 while preventing unintended destruction of the product, it is preferable to press the respective fiber sheets 11 and 12 with a relatively high pressure using the press roller 160 having a soft surface. Since a peripheral surface of the press roller can be made to follow the projections and depressions of the respective fiber sheets 11 and 12 generated by the absorbent polymer 13 when the respective fiber sheets 11 and 12 are pressed under such conditions, the press roller can efficiently pressurize the adhesives 15 applied onto the respective fiber sheets 11 and 12 so as to facilitate direct bonding in the microscopic voids. As a material of such a press roller 160, for example, hard rubber, silicon rubber, or silicon sponge can be used.

Finally, the long continuous absorbent sheet 1 is cut to a predetermined size by the sheet cutting unit to obtain the absorbent sheet of the present invention.

When the amount of the absorbent polymer 13 existing at both ends in one direction of the absorbent sheet 1 is smaller than the amount of the absorbent polymer in the central region, the spray amount of the absorbent polymer 13 is adjusted by the polymer spraying unit 150 such that the portions where the amount of the absorbent polymer 13 is small are formed at predetermined intervals in the conveyance direction R. Then, the position of the sheet cutting unit and the conveyance speed of the long continuous absorbent sheet may be adjusted so as to be cut at the portions. Thereby, the amount of the absorbent polymer 13 existing at both ends in the conveyance direction R of the absorbent sheet 1 is small. In a case of adjusting the amount of the absorbent polymer 13 existing in the peripheral region of the absorbent sheet 1, the amount of the absorbent polymer 13 sprayed on both ends of the second fiber sheet 12 and in the vicinity thereof in the direction orthogonal to the conveyance direction R may be adjusted in addition to the above-described method.

Through the above steps, the absorbent sheet of the present invention is produced. In the absorbent sheet, the absorbent polymer can sufficiently swell while being fixed at a predetermined position, and thus high liquid absorption performance can be exhibited.

Although the preferred embodiments of the present invention has been described above, the present invention is not limited to the above embodiments.

The following absorbent sheet, the absorbent article, and the producing method of the absorbent sheet will be disclosed with respect to the above-described embodiments of the present invention.

<1> An absorbent sheet comprising a first fiber sheet, a second fiber sheet, and an absorbent polymer disposed between the first sheet and the second fiber sheet, the first fiber sheet and the second sheet being joined to each other by an adhesive, wherein

in a region where the absorbent polymer is disposed, the absorbent polymer is disposed in such a manner that no macroscopically recognizable gap is observed, and
the region includes a portion where the first fiber sheet and the second sheet are directly joined to each other only by the adhesive without intervention of the absorbent polymer.

<2> The absorbent sheet as set forth in clause <1>, wherein the adhesive has elasticity.
<3> The absorbent sheet as set forth in clause <1> or <2>, wherein the absorbent sheet includes the portion where the first fiber sheet and the second sheet are directly joined to each other by the adhesive without intervention of the absorbent polymer and a portion where the first fiber sheet and the second sheet are joined to each other by the adhesive with intervention of the absorbent polymer.
<4> The absorbent sheet as set forth in any one of clauses <1> to <3>, wherein the portion where the first fiber sheet and the second sheet are directly joined to each other by the adhesive without intervention of the absorbent polymer is formed in plurality in a form of regularly or irregularly scattered dots when viewed in a sheet plane direction, and
a structure where projections and depressions are formed irregularly in scattered dots is preferable.
<5> The absorbent sheet as set forth in any one of clauses <1> to <4>, wherein the adhesive exists:

on a surface, of the first fiber sheet and the second sheet, which faces the absorbent polymer; and
in a void between fibers constituting the first fiber sheet and the second sheet.

<6> The absorbent sheet as set forth in any one of clauses <1> to <5>, wherein the adhesive is a rubber-based adhesive.

<7> The absorbent sheet as set forth in clause <6>, wherein the rubber-based adhesive is a styrene-based adhesive.

<8> The absorbent sheet as set forth in any one of clauses <1> to <7>, wherein the adhesive is a hot-melt adhesive.

<9> The absorbent sheet as set forth in any one of clauses <1> to <8>, wherein a relaxation time of the adhesive obtained by viscoelasticity measurement is 1 second or longer at 50°C.

<10> The absorbent sheet as set forth in any one of clauses <1> to <9>, wherein a relaxation time of the adhesive obtained by viscoelasticity measurement is preferably 2 seconds or longer, and more preferably 3 seconds or longer at 50°C, and

the relaxation time of the adhesive is preferably 20 seconds or shorter, more preferably 10 seconds or shorter, and even more preferably 5 seconds or shorter.

<11> The absorbent sheet as set forth in any one of clauses <1> to <10>, wherein the adhesive has a storage modulus G' at 25°C of preferably 10000 Pa or more, and more preferably 50000 Pa or more, and

the adhesive has a loss modulus G" at 25°C of preferably 10000 Pa or more, and more preferably 50000 Pa or more.

<12> The absorbent sheet as set forth in any one of clauses <1> to <11>, wherein the adhesive exists on a surface, of the first fiber sheet and the second sheet, which face the absorbent polymer and in a void between fibers constituting the first fiber sheet and the second sheet.

<13> The absorbent sheet as set forth in any one of clauses <1> to <12>, wherein an application area of the adhesive on the second fiber sheet is larger than an application area of the adhesive on the first fiber sheet, and

at a portion where the adhesive is applied, a first basis weight of the adhesive on the first fiber sheet is higher than a second basis weight of the adhesive on the second fiber sheet.

<14> The absorbent sheet as set forth in clause <13>, wherein a total of the first basis weight and the second basis weight is 500 g/m$^2$ or less.

<15> The absorbent sheet as set forth in clause <13> or <14>, wherein a total of the first basis weight and the second basis weight is preferably 500 g/m$^2$ or less, more preferably 300 g/m$^2$ or less, and even more preferably 200 g/m$^2$ or less,

the total of the first basis weight and the second basis weight is preferably 10 g/m$^2$ or more, more preferably 50 g/m$^2$ or more, and even more preferably 80 g/m$^2$ or more.

<16> The absorbent sheet as set forth in any one of clauses <13> to <15>, wherein the first basis weight is preferably 400 g/m$^2$ or less, more preferably 250 g/m$^2$ or less, and even more preferably 100 g/m$^2$, and

the first basis weight is practically 20g/m$^2$ or more.

<17> The absorbent sheet as set forth in any one of clauses <13> to <16>, wherein the second basis weight is preferably 30 g/m$^2$ or less, more preferably 15 g/m$^2$ or less, and even more preferably 10 g/m$^2$ or less, and

the second basis weight is practically 2 g/m$^2$ or more.

<18> The absorbent sheet as set forth in any one of clauses <1> to <17>, wherein the adhesive is discontinuously applied to a joined region of the first fiber sheet with the second fiber sheet so as to have a non-applied section,

the adhesive is continuously applied to an entire joined region of the second fiber sheet with the first fiber sheet so as not to have a gap, and

a surface of the first fiber sheet on which the adhesive does not exist is used as a liquid acquisition surface.

<19> The absorbent sheet as set forth in any one of clauses <1> to <18>, wherein a thickness of the adhesive existing on the surface of the first fiber sheet is thicker than a thickness of the adhesive existing on the surface of the second fiber sheet.

<20> The absorbent sheet as set forth in clause <19>, wherein the thickness of the adhesive existing on the surface of the first fiber sheet is thicker than the thickness of the adhesive existing on the surface of the second fiber sheet, and is preferably 20 μm or more, and more preferably 40 μm or more, and

the thickness of the adhesive existing on the surface of the first fiber sheet is preferably 500 μm or less, and more preferably 300 μm or less.

<21> The absorbent sheet as set forth in clause <19> or <20>, wherein the thickness of the adhesive existing on the surface of the second fiber sheet is preferably 1 μm or more, and more preferably 3 μm or more, and

the thickness of the adhesive existing on the surface of the second fiber sheet is preferably 20 μm or less, and more preferably 10 μm or less.

<22> The absorbent sheet as set forth in any one of clauses <1> to <21>, wherein a peeling strength between the first fiber sheet and the second sheet in a dry state is 0.1 N/25 mm or more and 5 N/25 mm or less.

<23> The absorbent sheet as set forth in any one of clauses <1> to <22>, wherein a peeling strength between the first fiber sheet and the second sheet in a dry state is preferably 0.1 N/25 mm or more, more preferably 0.2 N/25 mm or more, and even more preferably 0.3 N/25 mm or more, and

the peeling strength between the first fiber sheet and the second sheet in a dry state is preferably 3 N/25 mm or less, more preferably 2 N/25 mm or less, and even more preferably 1.5 N/25 mm or less.

<24> The absorbent sheet as set forth in any one of clauses <1> to <23>, wherein a thickness change is 1 mm or more during immersion in physiological saline for 30 minutes, and
the number of portions where the first fiber sheet and the second sheet are joined to each other is one or more per 1 cm$^2$ when both the fiber sheets are peeled off after being immersed.

<25> The absorbent sheet as set forth in any one of clauses <1> to <24>, wherein a thickness change is preferably 2 mm or more, more preferably 3 mm or more and even more preferably 4 mm or more during immersion in physiological saline for 30 minutes, and
the thickness change is practically 20 mm or less.

<26> The absorbent sheet as set forth in any one of clauses <1> to <25>, wherein when a state after the absorbent sheet in a dry state is immersed in physiological saline having an amount of 200 times a mass of the absorbent sheet for 30 minutes, then put on a net, and wiped off excess water with paper is a wet state, a compression strain ratio in the wet state is 25% or more.

<27> The absorbent sheet as set forth in clause <26>, wherein the compression strain ratio in the wet state is preferably 25% or more, more preferably 30% or more, and even more preferably 35% or more, and
the compression strain ratio is practically 70% or less.

<28> The absorbent sheet as set forth in clause <26> or <27>, wherein a swelling rate of the absorbent polymer is 80% or more, and
a peeling strength between the first fiber sheet and the second sheet in the wet state is 0.1 N/25 mm or more and 2 N/25 mm or less.

<29> The absorbent sheet as set forth in any one of clauses <26> to <28>, wherein a swelling rate of the absorbent polymer is preferably 80% or more, more preferably 85% or more, and even more preferably 90% or more.

<30> The absorbent sheet as set forth in any one of clauses <26> to <29>, wherein a peeling strength between the first fiber sheet and the second sheet in the wet state is preferably 0.1 N/25 mm or more, more preferably 0.2 N/25 mm or more, and even more preferably 0.3 N/25 mm or more, and
the peeling strength is preferably 2.0 N/25 mm or less, more preferably 1.5 N/25 mm or less, and even more preferably 1.0 N/25 mm or less.

<31> The absorbent sheet as set forth in any one of clauses <1> to <30>, wherein a surface of the first fiber sheet on which the adhesive does not exist has a projecting-and-depressed structure, and
the surface of the first fiber sheet on which the adhesive does not exist is used as a liquid acquisition surface.

<32> The absorbent sheet as set forth in any one of clauses <1> to <31>, wherein a basis weight of the absorbent polymer is 60 g/m$^2$ or more, and a median particle size of the absorbent polymer is 800 $\mu$m or less.

<33> The absorbent sheet as set forth in any one of clauses <1> to <32>, wherein an amount of the absorbent polymer existing in a central region is more than an amount of the absorbent polymer existing at both ends in one direction.

<34> The absorbent sheet as set forth in any one of clauses <1> to <33>, wherein an amount of the absorbent polymer existing in a central region is more than an amount of the absorbent polymer existing in a peripheral region.

<35> The absorbent sheet as set forth in any one of clauses <1> to <34>, wherein a liquid absorption amount of the absorbent polymer is preferably 15 g/g or more, more preferably 20 g/g or more, and even more preferably 25 g/g or more in a state where a load of 2.0 kPa is applied.

<36> The absorbent sheet as set forth in any one of clauses <1> to <35>, wherein the absorbent sheet further includes a fiber-entangling layer disposed adjacent to at least one of the first fiber sheet and the second sheet.

<37> The absorbent sheet as set forth in clause <36>, wherein at least one of the first fiber sheet and the second sheet adjacent to the fiber-entangling layer has a Klemm water absorption height of preferably 10 mm or higher, more preferably 20 mm or higher, and even more preferably 30 mm or higher.

<38> The absorbent sheet as set forth in any one of clauses <1> to <37>, wherein a basis weight of the first fiber sheet is preferably 6 g/m$^2$ or more, and more preferably 8 g/m$^2$ or more,
the basis weight of the first fiber sheet is preferably 50 g/m$^2$ or less, more preferably 30 g/m$^2$ or less, and even more preferably 20 g/m$^2$ or less.

<39> The absorbent sheet as set forth in any one of clauses <1> to <38>, wherein a basis weight of the second fiber sheet is preferably 4 g/m$^2$ or more, and more preferably 6 g/m$^2$ or more, and
the basis weight of the second fiber sheet is preferably 30 g/m$^2$ or less, and more preferably 20 g/m$^2$ or less.

<40> The absorbent sheet as set forth in any one of clauses <1> to <39>, wherein a thickness of the first fiber sheet is preferably 0.04 mm or more, and more preferably 0.08 mm or more, and
the thickness of the first fiber sheet is preferably 1 mm or less, and more preferably 0.3 mm or less.

<41> The absorbent sheet as set forth in any one of clauses <1> to <40>, wherein a thickness of the second fiber sheet is preferably 0.03 mm or more, and more preferably 0.07 mm or more, and
the thickness of the second fiber sheet is preferably 0.5 mm or less, and more preferably 0.2 mm or less.

<42> The absorbent sheet as set forth in any one of clauses <1> to <41>, wherein, Klemm water absorption height of the first fiber sheet is higher than Klemm water absorption height of the second fiber sheet, the Klemm water absorption height being measured according to JIS P8141.

<43> The absorbent sheet as set forth in any one of clauses <1> to <42>, wherein, air-permeability resistance of the second fiber sheet is lower than air-permeability resistance of the first fiber sheet, the air-permeability resistance being measured using a KES-F8 air-permeability tester.

<44> The absorbent sheet as set forth in any one of clauses <1> to <43>, wherein the first fiber sheet is paper having a crepe ratio of 5% or more, and

the second fiber sheet has a crepe ratio of less than 1%.

<45> The absorbent sheet as set forth in any one of clauses <1> to <44>, wherein an orientation propensity of fibers constituting the first fiber sheet matches an orientation propensity of fibers constituting the second fiber sheet.

<46> The absorbent sheet as set forth in any one of clauses <1> to <45>, wherein a thickness measured under a pressure of 1.7 kPa is preferably 0.3 mm or more, and more preferably 0.6 mm or more,

the thickness is preferably 4 mm or less, more preferably 3 mm or less, and even more preferably 2 mm or less.

<47> An absorbent article comprising the absorbent sheet as set forth in any one of clauses <1> to <46>.

<48> The absorbent article as set forth in clause <47>, wherein the first fiber sheet is disposed closer to a skin-facing surface, and

a surface of the first fiber sheet on which the adhesive does not exist has a projecting- and-depressed structure.

<49> A method for producing the absorbent sheet according to any one of claims 1 to 46, the method comprising:

applying the adhesive to each of one surface of the first fiber sheet and one surface of the second fiber sheet; spraying the absorbent polymer to an application surface of the adhesive on the second fiber sheet; and subsequently superposing the respective fiber sheets on each other such that application surfaces of the adhesive on the respective fiber sheets face each other.

Industrial Applicability

According to the present invention, there are provided an absorbent sheet and an absorbent article including the sheet in which an absorbent polymer can sufficiently swell while being fixed at a predetermined position and high liquid absorption performance can be exhibited.

## Claims

1. An absorbent sheet comprising a first fiber sheet, a second fiber sheet, and an absorbent polymer disposed between the first sheet and the second fiber sheet, the first fiber sheet and the second sheet being joined to each other by an adhesive, wherein

   in a region where the absorbent polymer is disposed, the absorbent polymer is disposed in such a manner that no macroscopically recognizable gap is observed, and
   the region includes a portion where the first fiber sheet and the second sheet are directly joined to each other only by the adhesive without intervention of the absorbent polymer.

2. The absorbent sheet according to claim 1, wherein the adhesive has elasticity.

3. The absorbent sheet according to claim 1 or 2, wherein the absorbent sheet includes the portion where the first fiber sheet and the second sheet are directly joined to each other by the adhesive without intervention of the absorbent polymer and a portion where the first fiber sheet and the second sheet are joined to each other by the adhesive with intervention of the absorbent polymer.

4. The absorbent sheet according to any one of claims 1 to 3, wherein the portion where the first fiber sheet and the second sheet are directly joined to each other by the adhesive without intervention of the absorbent polymer is formed in plurality in a form of regularly or irregularly scattered dots when viewed in a sheet plane direction.

5. The absorbent sheet according to any one of claims 1 to 4, wherein the adhesive exists:

   on a surface, of the first fiber sheet and the second sheet, which faces the absorbent polymer; and
   in a void between fibers constituting the first fiber sheet and the second sheet.

**6.** The absorbent sheet according to any one of claims 1 to 5, wherein the adhesive is a rubber-based adhesive.

**7.** The absorbent sheet according to of claim 6, wherein the rubber-based adhesive is a styrene-based adhesive.

**8.** The absorbent sheet according to any one of claims 1 to 7, wherein the adhesive is a hot-melt adhesive.

**9.** The absorbent sheet according to any one of claims 1 to 8, wherein a relaxation time of the adhesive obtained by viscoelasticity measurement is 1 second or longer at 50°C.

**10.** The absorbent sheet according to any one of claims 1 to 9, wherein a relaxation time of the adhesive obtained by viscoelasticity measurement is 2 seconds or longer at 50°C.

**11.** The absorbent sheet according to any one of claims 1 to 10, wherein the adhesive has a storage modulus G' at 25°C of 10000 Pa or more, and
the adhesive has a loss modulus G" at 25°C of 10000 Pa or more.

**12.** The absorbent sheet according to any one of claims 1 to 11, wherein the adhesive exists on a surface, of the first fiber sheet and the second sheet, which face the absorbent polymer and in a void between fibers constituting the first fiber sheet and the second sheet.

**13.** The absorbent sheet according to any one of claims 1 to 12, wherein

an application area of the adhesive on the second fiber sheet is larger than an application area of the adhesive on the first fiber sheet, and
at a portion where the adhesive is applied, a first basis weight of the adhesive on the first fiber sheet is higher than a second basis weight of the adhesive on the second fiber sheet.

**14.** The absorbent sheet according to of claim 13, wherein a total of the first basis weight and the second basis weight is 500 g/m$^2$ or less.

**15.** The absorbent sheet according to of claim 13 or 14, wherein a total of the first basis weight and the second basis weight is 10 g/m$^2$ or more and 300 g/m$^2$ or less.

**16.** The absorbent sheet according to any one of claims 13 to 15, wherein the first basis weight is 20 g/m$^2$ or more and 400 g/m$^2$ or less.

**17.** The absorbent sheet according to any one of claims 13 to 16, wherein the second basis weight is 2 g/m$^2$ or more and 30 g/m$^2$ or less.

**18.** The absorbent sheet according to any one of claims 1 to 17, wherein

the adhesive is discontinuously applied to a joined region of the first fiber sheet with the second fiber sheet so as to have a non-applied section,
the adhesive is continuously applied to an entire joined region of the second fiber sheet with the first fiber sheet so as not to have a gap, and
a surface of the first fiber sheet on which the adhesive does not exist is used as a liquid acquisition surface.

**19.** The absorbent sheet according to any one of claims 1 to 18, wherein a thickness of the adhesive existing on the surface of the first fiber sheet is thicker than a thickness of the adhesive existing on the surface of the second fiber sheet.

**20.** The absorbent sheet according to of claim 19, wherein the thickness of the adhesive existing on the surface of the first fiber sheet is 20 μm or more and 500 μm or less.

**21.** The absorbent sheet according to of claim 19 or 20, wherein the thickness of the adhesive existing on the surface of the second fiber sheet is 1 μm or more and 20 μm or less.

**22.** The absorbent sheet according to any one of claims 1 to 21, wherein a peeling strength between the first fiber sheet and the second sheet in a dry state is 0.1 N/25 mm or more and 5 N/25 mm or less.

23. The absorbent sheet according to any one of claims 1 to 22, wherein a peeling strength between the first fiber sheet and the second sheet in a dry state is 0.2 N/25 mm or more and 3 N/25 mm or less.

24. The absorbent sheet according to any one of claims 1 to 23, wherein a thickness change is 2 mm or more during immersion in physiological saline for 30 minutes, and
the number of portions where the first fiber sheet and the second sheet are joined to each other is one or more per 1 cm$^2$ when both the fiber sheets are peeled off after being immersed.

25. The absorbent sheet according to any one of claims 1 to 24, wherein a thickness change is 3 mm or more and 20 mm or less during immersion in physiological saline for 30 minutes.

26. The absorbent sheet according to any one of claims 1 to 25, wherein when a state after the absorbent sheet in a dry state is immersed in physiological saline having an amount of 200 times a mass of the absorbent sheet for 30 minutes, then put on a net, and wiped off excess water with paper is a wet state, a compression strain ratio in the wet state is 25% or more.

27. The absorbent sheet according to of claim 26, wherein the compression strain ratio in the wet state is 30% or more and 70% or less.

28. The absorbent sheet according to claim 26 or 27, wherein

    a swelling rate of the absorbent polymer is 80% or more, and
    a peeling strength between the first fiber sheet and the second sheet in the wet state is 0.1 N/25 mm or more and 2 N/25 mm or less.

29. The absorbent sheet according to any one of claims 26 to 28, wherein a swelling rate of the absorbent polymer is 85% or more.

30. The absorbent sheet according to any one of claims 26 to 29, wherein a peeling strength between the first fiber sheet and the second sheet in the wet state is 0.2 N/25 mm or more and 1.5 N/25 mm or less.

31. The absorbent sheet according to any one of claims 1 to 30, wherein

    a surface of the first fiber sheet on which the adhesive does not exist has a projecting- and-depressed structure, and
    the surface of the first fiber sheet on which the adhesive does not exist is used as a liquid acquisition surface.

32. The absorbent sheet according to any one of claims 1 to 31, wherein a basis weight of the absorbent polymer is 60 g/m$^2$ or more, and a median particle size of the absorbent polymer is 800 $\mu$m or less.

33. The absorbent sheet according to any one of claims 1 to 32, wherein an amount of the absorbent polymer existing in a central region is more than an amount of the absorbent polymer existing at both ends in one direction.

34. The absorbent sheet according to any one of claims 1 to 33, wherein an amount of the absorbent polymer existing in a central region is more than an amount of the absorbent polymer existing in a peripheral region.

35. The absorbent sheet according to any one of claims 1 to 34, wherein a liquid absorption amount of the absorbent polymer is 15 g/g or more in a state where a load of 2.0 kPa is applied.

36. The absorbent sheet according to any one of claims 1 to 35, further comprising a fiber-entangling layer disposed adjacent to at least one of the first fiber sheet and the second sheet.

37. The absorbent sheet according to of claim 36, wherein at least one of the first fiber sheet and the second sheet adjacent to the fiber-entangling layer has a Klemm water absorption height of 10 mm or higher.

38. The absorbent sheet according to any one of claims 1 to 37, wherein a basis weight of the first fiber sheet is 6 g/m$^2$

or more and 50 g/m$^2$ or less.

39. The absorbent sheet according to any one of claims 1 to 38, wherein a basis weight of the second fiber sheet is 4 g/m$^2$ or more and 30 g/m$^2$ or less.

40. The absorbent sheet according to any one of claims 1 to 39, wherein a thickness of the first fiber sheet is 0.04 mm or more and 1 mm or less.

41. The absorbent sheet according to any one of claims 1 to 40, wherein a thickness of the second fiber sheet is 0.03 mm or more and 0.5 mm or less.

42. The absorbent sheet according to any one of claims 1 to 41, wherein, Klemm water absorption height of the first fiber sheet is higher than Klemm water absorption height of the second fiber sheet, the Klemm water absorption height being measured according to JIS P8141.

43. The absorbent sheet according to any one of claims 1 to 42, wherein, air-permeability resistance of the second fiber sheet is lower than air-permeability resistance of the first fiber sheet, the air-permeability resistance being measured using a KES-F8 air-permeability tester.

44. The absorbent sheet according to any one of claims 1 to 43, wherein

the first fiber sheet is paper having a crepe ratio of 5% or more, and
the second fiber sheet has a crepe ratio of less than 1%.

45. The absorbent sheet according to any one of claims 1 to 44, wherein an orientation propensity of fibers constituting the first fiber sheet matches an orientation propensity of fibers constituting the second fiber sheet.

46. The absorbent sheet according to any one of claims 1 to 45, wherein a thickness measured under a pressure of 1.7 kPa is 0.3 mm or more and 4 mm or less.

47. An absorbent article comprising the absorbent sheet according to any one of claims 1 to 46.

48. The absorbent article according to of claim 47, wherein

the first fiber sheet is disposed closer to a skin-facing surface, and
a surface of the first fiber sheet on which the adhesive does not exist has a projecting- and-depressed structure.

49. A method for producing the absorbent sheet according to any one of claims 1 to 46, the method comprising:

applying the adhesive to each of one surface of the first fiber sheet and one surface of the second fiber sheet;
spraying the absorbent polymer to an application surface of the adhesive on the second fiber sheet; and
subsequently superposing the respective fiber sheets on each other such that application surfaces of the adhesive on the respective fiber sheets face each other.

EP 4 082 498 A1

Fig. 1

Fig. 2

Fig. 3

Fig. 4

<div align="center">

**INTERNATIONAL SEARCH REPORT**

</div>

| | International application No. |
|---|---|
| | PCT/JP2020/048177 |

| A. CLASSIFICATION OF SUBJECT MATTER |
|---|
| A61F 13/532(2006.01)i; A61F 13/534(2006.01)i |
| FI: A61F13/534 110; A61F13/532 210 |
| |
| According to International Patent Classification (IPC) or to both national classification and IPC |

| B. FIELDS SEARCHED |
|---|
| Minimum documentation searched (classification system followed by classification symbols) |
| A61F13/532; A61F13/534 |

| Documentation searched other than minimum documentation to the extent that such documents are included in the fields searched | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

| Electronic data base consulted during the international search (name of data base and, where practicable, search terms used) |
|---|
| |

| C. DOCUMENTS CONSIDERED TO BE RELEVANT |
|---|

| Category* | Citation of document, with indication, where appropriate, of the relevant passages | Relevant to claim No. |
|---|---|---|
| X | JP 2004-329664 A (LIVEDO CORP.) 25 November 2004 | 1 |
| Y | (2004-11-25) paragraphs [0018], [0027]-[0029], | 2-12, 22-35, |
| | [0057], fig. 6-9 | 38-49 |
| A | | 13-21, 36-37 |
| Y | JP 2017-176861 A (THE PROCTER & GAMBLE COMPANY) 05 October 2017 (2017-10-05) paragraph [0048] | 2-12, 22-35, 38-49 |
| Y | WO 2018/042544 A1 (UNI-CHARM CORP.) 08 March 2018 (2018-03-08) paragraph [0088], fig. 5 | 33-35, 38-49 |
| A | JP 2017-70500 A (KAO CORP.) 13 April 2017 (2017-04-13) | 1-49 |
| A | JP 2018-86148 A (KAO CORP.) 07 June 2018 (2018-06-07) | 37-49 |
| A | JP 2019-122717 A (DAIO PAPER CORP.) 25 July 2019 (2019-07-25) | 37-49 |

☒ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 March 2021 (11.03.2021) | 23 March 2021 (23.03.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

## INTERNATIONAL SEARCH REPORT

| International application No. |
| --- |
| PCT/JP2020/048177 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2018-79036 A (KAO CORP.) 24 May 2018 (2018-05-24) | 37-49 |

Form PCT/ISA/210 (continuation of second sheet) (January 2015)

INTERNATIONAL SEARCH REPORT

Information on patent family members

International application No.

PCT/JP2020/048177

| Patent Documents referred in the Report | Publication Date | Patent Family | Publication Date |
|---|---|---|---|
| JP 2004-329664 A | 25 Nov. 2004 | US 2007/0093164 A1 paragraphs [0029], [0038]-[0040], [0068], fig. 6-9 | |
| JP 2017-176861 A | 05 Oct. 2017 | EP 2740449 A1 paragraph [0046] | |
| WO 2018/042544 A1 | 08 Mar. 2018 | US 2019/0201247 A1 paragraph [0145], fig. 5 | |
| JP 2017-70500 A | 13 Apr. 2017 | US 2018/0296403 A1 | |
| JP 2018-86148 A | 07 Jun. 2018 | (Family: none) | |
| JP 2019-122717 A | 25 Jul. 2019 | (Family: none) | |
| JP 2018-79036 A | 24 May 2018 | (Family: none) | |

Form PCT/ISA/210 (patent family annex) (January 2015)

**EP 4 082 498 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002325799 A
- US 2012328861 A1
- JP 2018050987 A
- US 2007093164 A1
- JP 2019034229 A

**Non-patent literature cited in the description**

- Standardization and Analysis of Texture Evaluation. **SUEO KAWABATA.** Texture Measurement and Standardization Committee. The Textile Machinery Society of Japan, 10 July 1980